# EUROPÄISCHE PATENTSCHRIFT

(11) **EP 1 116 711 B1**
(45) Veröffentlichungstag und Bekanntmachung des Hinweises auf die Patenterteilung: **14.12.2005**
(21) Anmeldenummer: 00115071.3
(22) Anmeldetag: 27.07.2000
(51) Int. Cl.: C07C 211/51, A61K 7/13, C07D 215/38, C07D 307/52, C07D 295/12, C07D 241/04, C07D 307/12, C07C 233/36, C07C 239/20, C07C 215/14, C07C 217/08, C07C 215/76

(54) **2-Aminoalkyl-1,4-diaminobenzol-Derivate und diese Verbindungen enthaltende Färbemittel**
2-aminoalkyl-1,4-diaminobenzene derivatives and dye composition containing these compounds
Dérivés de 2-aminoalkyl-1,4-diaminobenzène et composition tinctoriale les contenant

(30) Priorität: 18.12.1999 DE 19961272
(43) Veröffentlichungstag der Anmeldung: 18.07.2001
(73) Patentinhaber: Wella Aktiengesellschaft, 64274 Darmstadt (DE)
(72) Erfinder: Chassot, Laurent, Dr., 1724 Praroman (CH); Braun, Hans-Jürgen, Dr., 3182 Überstorf (CH)

(56) Entgegenhaltungen:
- DE-C- 19 961 229
- DE-C- 19 961 274
- DE-U- 29 902 262
- US-A- 3 743 509
- US-A- 4 797 130

## Beschreibung

Die Erfindung betrifft Mittel zum Färben von Keratinfasern auf der Basis einer Entwicklersubstanz-Kupplersubstanz-Kombination, welche als Entwicklersubstanz 2-Aminoalkyl-1,4-diaminobenzol-Derivate enthalten, sowie neue 2-Aminoalkyl-1,4-diaminobenzol-Derivate.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol und Derivate des m-Phenylendiamins zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Mit den derzeit eingesetzten Färbemitteln, wie sie beispielsweise in der Monografie von K.H. Schrader "Grundlagen und Rezepturen der Kosmetika", 2. Aufl. (1989), Seiten 784-799 beschrieben werden, ist es jedoch nicht möglich, die vorgenannten Anforderungen in allen Punkten zu erfüllen. Es besteht daher weiterhin ein Bedürfnis nach neuen Entwicklersubstanzen, welche die vorgenannten Anforderung in besonderem Masse erfüllen.

US-A-4 797 130 offenbart ein Oxidationshaarfarbemittel auf der Basis von 4-Amino-2-aminomethyl-phenolen als Entwicklersubstanzen.

Hierzu wurde nun überraschenderweise gefunden, daß bestimmte 2-Aminoalkyl-1,4-diaminobenzol-Derivate gemäß der allgemeinen Formel (I) die an Entwicklersubstanzen gestellten Anforderungen in besonders hohem Maße erfüllen. So werden unter Verwendung dieser Entwicklersubstanzen mit den meisten bekannten Kupplersubstanzen farbstarke Farbnuancen erhalten, die außerordentlich lichtecht und waschecht sind.

Gegenstand der vorliegende Erfindung sind daher 2-Aminoalkyl-1,4-diaminobenzol-Derivate der allgemeinen Formel (I) 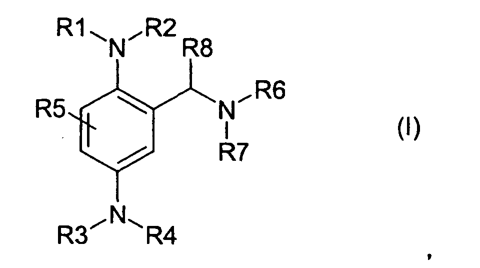 worin
**R1,R2,R3 und R4** unabhängig voneinander Wasserstoff, eine C₁-C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1und R2 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄-Hydroxyalkylgruppe oder einer C₁-C₄-Alkoxygruppe ist; **R6 und R7** unabhänging voneinander gleich Wasserstoff, einer C₁-C₂-Alkoxygruppe, einer C₁-C₆-Alkylgruppe, einer ungesättigten C₁-C₆-Alkylgruppe, einer C₁-C₄-Hydroxyalkylgruppe, einer C₃-C₄-Dihydroxyalkylgruppe, einer C₁-C₄-Aminoalkylgruppe, einer C₁-C₄-Dimethylaminoalkylgruppe, einer C₁-C₄-Acetylaminoalkylgruppe einer C₁-C₄-Methoxyalkylgruppe, einer C₁-C₄-Ethoxyalkylgruppe, einer C₁-C₄-Cyanalkylgruppe, einer C₁-C₄-Carboxyalkylgruppe, einer C₁-C₄-Aminocarbonylalkylgruppe, einer Pyridylmethylgruppe, einer Furfurylgruppe, einer hydrierten Furfurylgruppe, einer substituierten Pyridylgruppe, einem Rest der Formel (II) 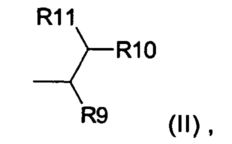 einem Rest der Formel (III) 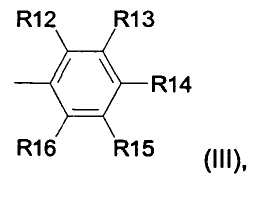 einem Rest der Formel (IV) 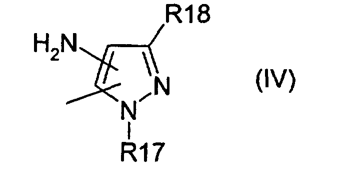 sind oder R6 und R7 einen Ring der Formel 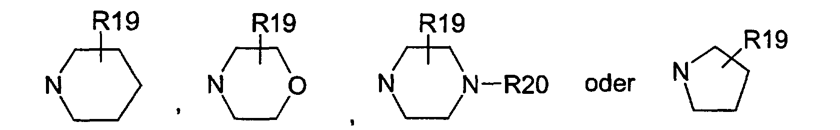 bilden, wobei mindestens einer der Reste R6, R7 kein Wasserstoff ist;
**R8** gleich Wasserstoff, oder einer C₁-C₆ Alkylgruppe Gruppe ist;
**R9** gleich Wasserstoff, einer Carboxygruppe, oder einer Aminocarbonylgruppe ist;
**R10, R11** unabhänging voneinander gleich Wasserstoff, einer Hydroxygruppe, einer Aminocarbonylgruppe, einer Methylthiomethylgruppe, einem mit einer Phenylgruppe oder Hydroxygruppe substituierten Phenylrest oder einem Rest der Formel 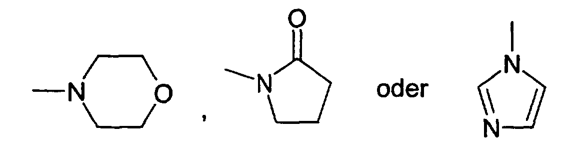 ist ,
**R12,R13,R14,R15,R16** unabhängig voneinander Wasserstoff, ein Halogenatom, eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄-Alkylthioethergruppe, eine Mercaptogruppe, eine Nitrogruppe, eine Aminogruppe, eine Alkylaminogruppe, eine Hydroxyalkylaminogruppe, eine Dialkylaminogruppe, eine Di(hydroxyalkyl)aminogruppe, eine (Dihydroxyalkyl)aminogruppe, eine (Hydroxyalkyl)alkylaminogruppe, eine Trifluormethan-gruppe, eine -C(O)H-Gruppe, eine -C(O)CH₃-Gruppe, eine -C(O)CF₃-Gruppe, eine -Si(CH₃)₃-Gruppe, eine C₁-C₄-Hydroxyalkylgruppe, eine C₃-C₄ Dihydroxyalkylgruppe bedeuten, oder zwei nebeneinanderliegende Reste R12 bis R16 eine -O-CH2-O-Brücke bilden;
**R17** gleich einer C₁-C₄-Alkylgruppe oder einer C₁-C₄-Hydroxyalkylgruppe ist;
**R18** gleich Wasserstoff oder einer C₁-C₆ Alkylgruppe Gruppe ist;
**R19** gleich einer oder mehreren Wasserstoff, oder Hydroxy-, Carboxy-, Aminocarbonyl-, oder Hydroxymethylgruppe ist;
**R20** gleich Wasserstoff, oder einer C₁-C₆ Alkylgruppe Gruppe ist; oder deren physiologisch verträgliche, wasserlösliche Salze enthalten.

Als Verbindungen der Formel (I) können beispielweise die folgenden Verbindungen genannt werden:
2-(2,3-Dihydroxypropyl)aminomethyl-1,4-diamino-benzol; 2-Ethylamino-methyl-1,4-diamino-benzol; 2-(Isopropylamino-methyl)-1,4-diamino-benzol; 2-Propylaminomethyl-1,4-diamino-benzol; 2-Pyrrolidin-1-ylmethyl-1,4-diamino-benzol; 2-[(2-Methoxy-ethylamino)-methyl]-1,4-diamino-benzol; 2-Morpholin-4-ylmethyl-1,4-diamino-benzol; 2-(2,5-Diamino-benzylamino)-butan-1-ol; 2-{[(Furan-2-ylmethyl)-amino]-methyl}-1,4-diamino-benzol; N-(2,5-Diamino-benzyl)-O,N-dimethyl-hydroxylamin; 2-(4-Methyl-piperazin-1-ylmethyl)-1,4-diamino-benzol; 1-(2,5-Diamino-benzyl)-piperidin-4-ol; N-[2-(2,5-Diamino-benzylamino)-ethyl]-acetamid; 2-[(2-Morpholin-4-yl-ethylamino)-methyl]-1,4-diamino-benzol; 2-Allylaminomethyl-1,4-diamino-benzol; 2-(2,5-Diamino-benzylamino)-propan-1-ol; 2-[(3-Imidazol-1-yl-propylamino)-methyl]-1,4-diamino-benzol; 2-{[(Tetrahydro-furan-2-ylmethyl)-amino]-methyl}-1,4-diamino-benzol; 4-(2,5-Diamino-benzylamino)-anilin; 3-(2,5-Diamino-benzylamino)-phenol; 5-(2,5-Diamino-benzylamino)-2-methyl-phenol; 2-[(2-Dimethylamino-ethylamino)-methyl]-1,4-diamino-benzol; 4-(2,5-Diamino-benzylamino)-butan-1-ol; 2-[(3-Ethoxy-propylamino)-methyl]-1,4-diamino-benzol; 2-[(3-Methoxy-phenylamino)-methyl]-1,4-diamino-benzol; 2-[(4-Chlor-phenylamino)-methyl]-1,4-diamino-benzol; 2-[(Cyclopropylmethyl-amino)-methyl]-1,4-diamino-benzol; 2-(2,5-Diamino-benzylamino)-4-nitro-phenol; 2-[(4-Chlor-benzylamino)-methyl]-1,4-diamino-benzol; 2-[(2,5-Diamino-benzyl)-methyl-amino]-ethanol; 2-[(2,5-Diamino-benzyl)-ethyl-amino]-ethanol; 2-{[(Pyridin-4-ylmethyl)-amino]-methyl}-1,4-diamino-benzol; 1-[3-(2,5-Diamino-benzylamino)-propyl]-pyrrolidin-2-on; 2-(4-Amino-2 methyl-phenyl)aminomethyl-1,4-diamino-benzol; 2-(4-Amino-3 methyl-phenyl)aminomethyl-1,4-diamino-benzol; 2-[5-Amino-2-(2,5-diamino-benzylamino)-phenyl]-ethanol; 2-(3-Amino-phenyl)aminomethyl-1,4-diamino-benzol; 4-[2-(2,5-Diamino-benzylamino)-ethyl]-benzolsulfonamid; 2-[4-Amino-2-(2,5-diamino-benzylamino)-phenoxy]-ethanol; 2-[(2,5-Diamino-benzyl)-(2-hydroxy-ethyl)-amino]-ethanol; [1-(2,5-Diamino-benzyl)-pyrrolidin-2-yl]-methanol; 1-(2,5-Diamino-benzyl)-pyrrolidin-3-ol; 1-(2,5-Diamino-benzyl)-pyrrolidin-2-carbonsäureamid; 1-(2,5-Diamino-benzyl)-piperidin-3-ol; 2-(2,5-Diamino-benzylamino)-propan-1,3-diol; 2-(2,5-Diamino-benzylamino)-3-hydroxy-propionamid; 2-(2,5-Diamino-benzylamino)-bernsteinsäure; 2-Cyclopropylaminomethyl-1,4-diamino-benzol; 2-(2,5-Diamino-benzylamino)-ethanol; (2,5-Diamino-benzylamino)-essigsäure; 4-(2,5-Diamino-benzylamino)-phenol; 2-(Benzo[1,3]dioxol-5-ylaminomethyl)-1,4-diamino-benzol; [(2,5-Diamino-benzyl)-methyl-amino]-acetonitril; 2-Pentylaminomethyl-1,4-diamino-benzol; 2-[(3-Dimethyl-amino-propylamino)-methyl]-1,4-diamino-benzol; 2-{[2-(5-Nitro-pyridin-2-ylamino)-ethylamino]-methyl}-1,4-diamino-benzol; 2-[(2-Amino-ethylamino)-methyl]-1,4-diamino-benzol; 3-[2-(2,5-Diamino-benzylamino)-1-hydroxy-ethyl]-phenol; 2-[(4-Methyl-pyridin-2-ylamino)-methyl]-1,4-diamino-benzol; 2-(2,5-Diamino-benzyl)-1-methyl-1,2,3,4-tetrahydroisochinolin-6,7-diol; 2-(2,5-Diamino-benzylamino)-4-methylsulfanylbuttersäure; 1-(2,5-Diamino-benzyl)-pyrrolidin-2-carbonsäure; 2-Phenylaminomethyl-1,4-diamino-benzol; 2-(4-Dimethylamino-phenylaminomethyl-1,4-diamino-benzol; 1-[3-(2,5-Diamino-benzylamino)-phenyl]-ethanol; 1-[4-(2,5-Diamino-benzylamino)-phenyl]-ethanol; 1-[2-(2,5-Diamino-benzylamino)-phenyl]-ethanol; 2-[(3,4-Dimethoxy-phenylamino)-methyl]-1,4-diamino-benzol; 2-[(3-Fluor-2-methoxy-phenylamino)-methyl]-1,4-diamino-benzol; 4-Chlor-2-(2,5-diamino-benzylamino)-phenol; 2-[(4-Trifluoromethyl-phenylamino)-methyl]-1,4-diamino-benzol; 2-(p-Tolylamino-methyl)-1,4-diamino-benzol; N⁴-Dihydroxypropyl-2-(di(hydroxyethyl)amino-methyl)-1,4-diamino-benzol; N⁴-Dihydroxypropyl-2-(hydroxyethylamino-methyl)-1,4-diamino-benzol; N⁴-Dihydroxypropyl-2-(methylamino-methyl)-1,4-diamino-benzol; N⁴-Hydroxyethyl-2-(di(hydroxyethyl)amino-methyl)-1,4-diamino-benzol; N⁴-Hydroxyethyl-2-(hydroxyethylamino-methyl)-1,4-diamino-benzol; N⁴-Hydroxyethyl-2-(methylamino-methyl)-1,4-diamino-benzol; N⁴,N⁴-Bis(hydroxyethyl)-2-(di(hydroxyethyl)amino-methyl)-1,4-diamino-benzol; N⁴,N⁴-Bis(hydroxyethyl)-2-(hydroxyethylamino-methyl)-1,4-diamino-benzol; N⁴,N⁴-Bis(hydroxyethyl)-2-(methylamino-methyl)-1,4-diamino-benzol; N¹-Dihydroxypropyl-2-(di(hydroxyethyl)amino-methyl)-1,4-diamino-benzol; N¹-Dihydroxypropyl-2-(hydroxyethylamino-methyl)-1,4-diamino-benzol; N¹-Dihydroxypropyl-2-(methylamino-methyl)-1,4-diamino-benzol; N¹-Hydroxyethyl-2-(di(hydroxyethyl)amino-methyl)-1,4-diamino-benzol; N¹-Hydroxyethyl-2-(hydroxyethylamino-methyl)-1,4-diamino-benzol; N¹-Hydroxyethyl-2-(methylamino-methyl)-1,4-diamino-benzol; N¹,N¹-Bis(hydroxyethyl)-2-(di(hydroxyethyl)amino-methyl)-1,4-diamino-benzol; N¹,N¹-Bis(hydroxyethyl)-2-(hydroxyethylamino-methyl)-1,4-diamino-benzol; N¹,N¹-Bis(hydroxyethyl)-2-(methylamino-methyl)-1,4-diamino-benzol; 2-((2-Amino-phenylamino)-methyl)-1,4-diamino-benzol; 2-((2-Hydroxy-phenylamino)-methyl)-1,4-diamino-benzol; 2-((2-Dimethylamino-phenylamino)-methyl)-1,4-diamino-benzol; 2-((2-Methyl-phenylamino)-methyl)-1,4-diamino-benzol; 2-((2-Trifluoromethyl-phenylamino)-methyl)-1,4-diamino-benzol; 2-((3-Dimethylamino-phenylamino)-methyl)-1,4-diamino-benzol; 2-((3-Methyl-phenylamino)-methyl)-1,4-diamino-benzol; 2-((3-Trifluoromethyl-phenylamino)-methyl)-1,4-diamino-benzol; 2-((2-Brom-phenylamino)-methyl)-1,4-diamino-benzol; 2-((2-Cyano-phenylamino)-methyl)-1,4-diamino-benzol; 2-((2-Fluoro-phenylamino)-methyl)-1,4-diamino-benzol; 2-((2-Methoxy-phenylamino)-methyl)-1,4-diamino-benzol; 2-((2-Nitro-phenylamino) methyl)-1,4-diamino-benzol; 2-((3-Brom-phenylamino)-methyl)-1,4-diamino-benzol; 2-((3-Cyano-phenylamino)-methyl)-1,4-diamino-benzol; 2-((3-Fluor-phenylamino)-methyl)-1,4-diamino-benzol; 2-((3-Methoxy-phenylamino)-methyl)-1,4-diamino-benzol; 2-((3-Nitro-phenylamino)-methyl)-1,4-diamino-benzol; 2-((4-Brom-phenylamino)-methyl)-1,4-diamino-benzol; 2-((4-Cyano-phenylamino)-methyl)-1,4-diamino-benzol; 2-((4-Fluor-phenylamino)-methyl)-1,4-diamino-benzol; 2-((4-Methoxy-phenylamino)-methyl)-1,4-diamino-benzol; 2-((4-Nitro-phenylamino) methyl)-1,4-diamino-benzol; 2-((2-(1,3-Dihydroxypropyl)amino-phenylamino)-methyl)-1,4-diamino-benzol; 2-((2-Di(hydroxyethyl)amino-phenylamino)-methyl)-1,4-diamino-benzol; 2-((2-Hydroxyethylamino-phenylamino)-methyl)-1,4-diamino-benzol; 2-((2-Pyrrolidin-phenylamino)-methyl)-1,4-diamino-benzol; 2-((3-(1,3-Dihydroxypropyl)amino-phenylamino)-methyl)-1,4-diamino-benzol; 2-( (3-Di(hyd roxyethyl)amino-phenylamino)-methyl)-1,4-diamino-benzol; 2-((3-Hydroxyethylamino-phenylamino)-methyl)-1,4-diamino-benzol; 2-((3-Pyrrolidin-phenylamino)-methyl)-1,4-diamino-benzol; 2-((4-(1,3-Dihydroxypropyl)amino-phenylamino)-methyl)-1,4-diamino-benzol; 2-((4-Di(hydroxyethyl)amino-phenylamino)-methyl)-1,4-diamino-benzol; 2-((4-Hydroxyethylamino-phenylamino)-methyl)-1,4-diamino-benzol; 2-((4-Pyrrolidin-phenylamino) methyl)-1,4-diamino-benzol; 2-((4-Amino-2-(2-hydroxyethoxy) phenylamino)-methyl)-1,4-diamino-benzol; 2-((4-Amino-2-chlor-phenylamino)-methyl)-1,4-diamino-benzol; 2-((4-Amino-2-hydroxy-phenylamino)-methyl)-1,4-diamino-benzol; 2-((4-Amino-2-methoxy-phenylamino)-methyl)-1,4-diamino-benzol; 2-((4-Amino-3-(2-hydroxyethoxy)-phenylamino)-methyl)-1,4-diamino-benzol; 2-((4-Amino-3-chloro-phenylamino)-methyl)-1,4-diamino-benzol; 2-((4-Amino-3-hydroxy-phenylamino)-methyl)-1,4-diamino-benzol; 2-((4-Amino-3-methoxy-phenylamino)-methyl)-1,4-diamino-benzol; 2-((3,4-Diamino-phenylamino)-methyl)-1,4-diamino-benzol; 2-((2,4-Diamino-phenylamino)-methyl)-1,4-diamino-benzol; N⁴-Dihydroxypropyl-2-((4-amino-phenylamino)-methyl)-1,4-diamino-benzol; N⁴-Dihydroxypropyl-2-(phenylamino-methyl)-1,4-diamino-benzol; N⁴-Dihydroxypropyl-2-((4-di(hydroxyethyl)amino-phenylamino)-methyl)-1,4-diamino-benzol; N⁴-Dihydroxypropyl-2-((4-hydroxyethylamino-phenylamino)-methyl)-1,4-diamino-benzol; N⁴-Hydroxyethyl-2-((4-amino-phenylamino)-methyl)-1,4-diamino-benzol; N⁴-Hydroxyethyl-2-(phenylamino-methyl)-1,4-diamino-benzol; N⁴-Hydroxyethyl-2-((4-di(hydroxyethyl)amino-phenylamino)-methyl)-1,4-diamino-benzol; N⁴-Hydroxyethyl-2-((4-hydroxyethylamino-phenylamino) methyl)-1,4-diamino-benzol; N⁴,N⁴-Bis(hydroxyethyl)-2-(4-amino-phenylamino)-methyl)-1,4-diamino-benzol; N⁴,N⁴-Bis(hydroxyethyl)-2-phenylamino-methyl)-1,4-diamino-benzol; N⁴,N⁴-Bis(hydroxyethyl)-2-(4-di(hydroxyethyl)amino-phenylamino)-methyl)-1,4-diamino-benzol; N⁴,N⁴-Bis(hydroxyethyl)-2-(4-hydroxyethylamino-phenylamino)-methyl)-1,4-diamino-benzol; N¹-Dihydroxypropyl-2-((4-amino-phenylamino) methyl)-1,4-diamino-benzol; N¹-Dihydroxypropyl-2-(phenylamino-methyl)-1,4-diamino-benzol; N¹-Dihydroxypropyl-2-((4-di(hydroxyethyl)amino-phenylamino)-methyl)-1,4-diamino-benzol; N¹-Dihydroxypropyl-2-((4-hydroxyethylamino-phenylamino)-methyl)-1,4-diamino-benzol; N¹-Hydroxyethyl-2-((4-amino-phenylamino)-methyl)-1,4-diamino-benzol; N¹-Hydroxyethyl-2-(phenylamino-methyl)-1,4-diamino-benzol; N¹-Hydroxyethyl-2-(4-di(hydroxyethyl)amino-phenylamino)-methyl)-1,4-diamino-benzol; N¹-Hydroxyethyl-2-((4-hydroxyethylamino-phenylamino) methyl)-1,4-diamino-benzol; N¹,N¹-Bis(hydroxyethyl)-2-(4-amino-phenylamino)-methyl)-1,4-diamino-benzol; N¹,N¹-Bis(hydroxyethyl)-2-phenylamino-methyl)-1,4-diamino-benzol; N¹,N¹-Bis(hydroxyethyl)-2-(4-di(hydroxyethyl)amino-phenylamino)-methyl)-1,4-diamino-benzol; N¹,N¹-Bis(hydroxyethyl)-2-(4-hydroxyethylamino-phenylamino)-methyl)-1,4-diamino-benzol; 2-[5-Amino-4-(2,5-diamino-phenylamino)-pyrazol-1-yl]-ethanol; N2-(5-Amino-1-methyl-1H-pyrazol-4-yl)-1,2,4-triamino-benzol; N2-(5-Amino-1-isopropyl-1 H-pyrazol-4-yl)-1,2,4-triamino-benzol und N2-(5-Amino-1,3-dimethyl-1 H-pyrazol-4-yl)-1,2,4-triamino-benzol.

Bevorzugt sind Verbindungen der Formel (I), bei denen (i) eine oder mehrere der Restgruppen R5 und R8 gleich Wasserstoff sind und/oder (ii) R1, R2, R3 und R4 gleichzeitig Wasserstoff bedeuten und/oder (iii) R6 gleich einer Methylgruppe oder einer C₁-C₄-Hydroxyalkylgruppe und R7 gleich einer C₁-C₄-Hydroxyalkylgruppe ist und/oder (iv) R6 gleich Wasserstoff und R7 gleich einer C₁-C₄-Hydroxyalkylgruppe, einem substituierten Pyridylrest, einem substituierten Phenylrest, einem subsituierten Pyrazoylrest oder einem Rest der folgenden Formel 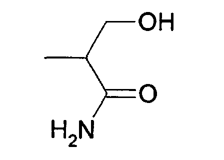 ist, und/oder (v) R6 und R7 einen aliphatischen Ring der Formel 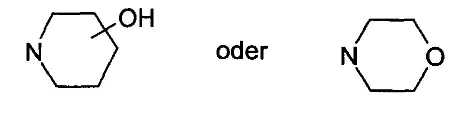 bilden.

Insbesondere sind die folgenden Verbindungen zu nennen: 2-(2,3-Dihydroxypropyl)aminomethyl-1,4-diamino-benzol; 2-[(2-amino-ethylamino)-methyl]-1,4-diamino-benzol; 2-[(2-hydroxyethylamino)-methyl]-1,4-diamino-benzol; 2-[(2,5-Diamino-benzyl)-methyl-amino]-ethanol; 2-(2,5-Diamino-benzylamino)-propan-1-ol; 2-[(2,5-Diamino-benzyl)-(2-hydroxy-ethyl)-amino]-ethanol; [1-(2,5-Diamino-benzyl) pyrrolidin-2-yl]-methanol; 1-(2,5-Diamino-benzyl)-pyrrolidin-2-carbonsäureamid; 2-[(4-Methyl-pyridin-2-ylamino)-methyl]-1,4-diamino-benzol; 2-((2-Amino-phenylamino)-methyl)-1,4-diamino-benzol; 2-((2-Chlor-phenylamino)-methyl)-1,4-diamino-benzol; 2-((2-Dimethylamino-phenylamino)-methyl)-1,4-diamino-benzol; 2-((2-Fluor-phenylamino)-methyl)-1,4-diamino-benzol; 2-((2-Hydroxyethylamino-phenylamino)-methyl)-1,4-diamino-benzol; 2-((2-N,N-Bis(hydroxyethyl)-amino-phenylamino)-methyl)-1,4-diamino-benzol; 2-((2-Pyrrolidin-phenylamino)-methyl)-1,4-diamino-benzol; 2-((3-Amino-phenylamino) methyl)-1,4-diamino-benzol; 2-((3-Chlor-phenylamino)-methyl)-1,4-diamino-benzol; 2-((3-Dimethylamino-phenylamino)-methyl)-1,4-diamino-benzol; 2-((3-Fluor-phenylamino)-methyl)-1,4-diamino-benzol; 2-((3-Hydroxyethylamino-phenylamino)-methyl)-1,4-diamino-benzol; 2-((3-N,N-Bis(hydroxyethyl)amino-phenylamino)-methyl)-1,4-diamino-benzol; 2-((3-Pyrrolidin-phenylamino)-methyl)-1,4-diamino-benzol; 2-((4-Amino-phenylamino)-methyl)-1,4-diamino-benzol; 2-((4-Chlor-phenylamino)-methyl)-1,4-diamino-benzol; 2-((4-Dimethylamino-phenylamino)-methyl)-1,4-diamino-benzol; 2-((4-Fluor-phenylamino)-methyl)-1,4-diamino-benzol; 2-((4-Hydroxyethylamino-phenylamino)-methyl)-1,4-diamino-benzol; 2-((4-N,N-Bis(hydroxyethyl)amino-phenylamino)-methyl)-1,4-diamino-benzol; 2-((4-Pyrrolidin-phenylamino)-methyl)-1,4-diamino-benzol; 2-((2-(2-Hydroxy)-ethoxy-4-amino-phenylamino)-methyl)-1,4-diamino-benzol; 2-((2-Amino-4-amino-phenylamino)-methyl)-1,4-diamino-benzol; 2-((2-Chlor-4-amino-phenylamino)-methyl)-1,4-diamino-benzol; 2-((2-Hydroxy-4-amino-phenylamino)-methyl)-1,4-diamino-benzol; 2-((2-Hydroxyethylamino-4-amino-phenylamino)-methyl)-1,4-diamino-benzol; 2-((2-Methyl-4-amino-phenylamino)-methyl)-1,4-diamino-benzol; 2-((3-(2-Hydroxy)-ethoxy-4-amino-phenylamino)-methyl)-1,4-diamino-benzol; 2-((3-Amino-4-amino-phenylamino)-methyl)-1,4-diamino-benzol; 2-((3-Chlor-4-amino-phenylamino)-methyl)-1,4-diamino-benzol; 2-((3-Hydroxy-4-amino-phenylamino)-methyl)-1,4-diamino-benzol; 2-((3-Hydroxyethylamino-4-amino-phenylamino)-methyl)-1,4-diamino-benzol; 2-((3-Methyl-4-amino-phenylamino)-methyl)-1,4-diamino-benzol; 2-((2-(2-Hydroxy)-ethoxy-phenylamino)-methyl)-1,4-diamino-benzol; 2-((2-Hydroxy-phenylamino)-methyl)-1,4-diamino-benzol; 2-((3-(2-Hydroxy)-ethoxy-phenylamino) methyl)-1,4-diamino-benzol; 2-((3-Hydroxy-phenylamino)-methyl)-1,4-diamino-benzol; 2-((4-(2-Hydroxy)-ethoxy-phenylamino)-methyl)-1,4-diamino-benzol; 2-((4-Hydroxy-phenylamino)-methyl)-1,4-diamino-benzol; 2-(Phenylamino)-methyl)-1,4-diamino-benzol; 2-[5-Amino-4-(2,5-diamino-phenylamino)-pyrazol-1-yl]-ethanol; N²-(5-Amino-1-methyl-1H-pyrazol-4-yl)-1,2,4-triamino-benzol; N²-(5-Amino-1-isopropyl-1H-pyrazol-4-yl)-1,2,4-triamino-benzol und N²-(5-Amino-1,3-dimethyl-1H-pyrazol-4-yl)-1,2,4-triamino-benzol.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen Diaminobenzol-Derivate der Formel (I) kann unter Verwendung von bekannten Syntheseverfahren erfolgen. Die Synthese der erfindungsgemäßen Verbindungen kann beispielsweise wie folgt durchgeführt werden:
Entweder a) durch eine reduktive Aminierung eines substituierten Benzols der Formel (V) 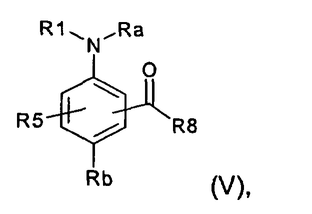 worin Ra für eine Schutzgruppe, wie sie zum Beispiel in dem Kapitel "Protective Groups" in Organic Synthesis, Kapitel 7, Wiley Interscience, 1991 beschrieben wird, steht; Rb die Bedeutung NR1Ra oder NR1R2 hat, mit einem Amin der Formel HNR6R7, wobei R1, R2, R5, R6, R7 und R8 die in Formel (I) genannte Bedeutung haben, und anschließende Abspaltung der Schutzgruppe;
oder b) durch Substition eines substituierten Benzols der Formel (VI) 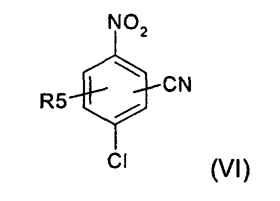 mit einem Amin der Formel HNR1 R2, Reduktion der Nitrilgruppe, anschliessende Alkylierung der Aminogruppe mit einer Verbindung der Formel XR6 und/oder XR7, und abschliessende Reduktion der Nitrogruppe, wobei R1, R2, R5, R6 und R7 die in Formel (I) angegebene Bedeutung haben und X gleich einem Halogenatom ist.

Die erfindungsgemäßen 2-Aminoalkyl-1,4-diaminobenzol-Derivate der Formel (I) sind in Wasser gut löslich und ermöglichen Färbungen mit hoher Farbintensität und ausgezeichneter Farbechtheit, insbesondere was die Lichtechtheit, Waschechtheit und Reibeechtheit anbetrifft. Die 2-Aminoalkyl-1,4-diaminobenzol-Derivate der Formel (I) weisen weiterhin eine ausgezeichnete Lagerstabilität, insbesondere als Bestandteil der nachfolgend beschriebenen Färbemittel, auf.

Ein weiterer Gegenstand der vorliegenden Erfindung sind daher Mittel zum oxidativen Färben von Keratinfasern, wie zum Beispiel Haaren, Pelzen, Federn oder Wolle, insbesondere menschlichen Haaren, auf der Basis einer Entwicklersubstanz-Kupplersubstanz-Kombination, welche als Entwicklersubstanz mindestens ein 2- Aminoalkyl-1,4-diaminobenzol-Derivat der Formel (I) enthalten.

Das 2-Aminoalkyl-1,4-diaminobenzol-Derivat der Formel (I) ist in dem erfindungsgemäßen Färbemittel in einer Menge von etwa 0,005 bis 20 Gewichtsprozent enthalten, wobei eine Menge von etwa 0,01 bis 8,0 Gewichtsprozent und insbesondere 0,1 bis 5,0 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol,7-Hydroxy-indol und 2,3-Indolindion in Betracht.

Obwohl die vorteilhaften Eigenschaften der hier beschriebenen 2-Aminoalkyl-1,4-diaminobenzol-Derivate der Formel (I) es nahelegen, diese als alleinige Entwicklersubstanz zu verwenden, ist es selbstverständlich auch möglich, die 2-Aminoalkyl-1,4-diaminobenzol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diaminopyrazol-Derivaten wie zum Beispiel 4,5-Diamino-1-(2-hydroxyethyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"-cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein. Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,8 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Haarfärbemittel mit einem Gehalt an 2-Aminoalkyl-1,4-diaminobenzol-Derivaten der Formel (I) als Entwicklersubstanz ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, welche sich von blonden über braune, purpure, violette bis hin zu blauen und schwarzen Farbtönen erstreckt. Hierbei zeichnen sich die Farbtöne durch ihre besondere Farbintensität aus. Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Die nachfolgenden Beispiele sollen den Gegenstand der Erfindung näher erläutern, ohne ihn darauf zu beschränken.

### Beispiele

### Beispiele 1: Synthese von 2,5-Diamino-1-aminomethyl-benzol-Derivaten der Formel (I) (Allgemeine Synthesevorschrift)

### A. Synthese von 2,5-Bis-tert.-butyloxycarbonylamino-brombenzol

15,65g (0,07 mol) Brom-p-phenylendiamin-Hydrochlorid und 32,7 g (0,15 mol) Di-tert.-butyl-dicarbonat werden in einer Mischung von 250 ml 2N Natriunhydroxide und 250 ml Trifluortoluol gelöst und auf 45 °C erwärmt. Die Reaktionmischung wird 3 Tage gerührt. Schrittweise werden noch insgesamt 30 g (0,14 mol) Di-tert.-butyl-dicarbonat zugegeben. Anschließend wird die organische Schicht abgetrennt und die wäßrige Phase noch zweimal mit 100ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-Bis-tert.-butyloxycarbonylamino-brombenzol mit einem Schmelzpunkt von 130 °C erhalten.

### B. Synthese von N-(4-tert.Butyloxycarbonylamino-2-formyl-phenyl)-carbaminsäure-tert.butylester

3,3 g (0,01 mol) 2,5-Bis-tert.-butyloxycarbonylamino-brombenzol aus Stufe **A** werden unter Argon in 100 ml wasserfreiem Tetrahydrofuran gelöst. Schrittweise werden 17 ml einer 1,6 molaren etherischen Methyllithiumlösung (0,03 mol) zugegeben. Die Reaktionsmischung wird auf -20 °C gekühlt, 7 ml einer 1,5 molaren tert.-Butyllithiumlösung (0,01 mol) werden noch schrittweise zugegeben. Nach beendeter Zugabe wird die Lösung noch 30 Minuten bei der angegebenen Temperatur gerührt. Anschliessend werden 1,2 g Dimethylformamid (0,02 mol) zugegeben und die Reaktionmischung wird eine Stunde bei -20 °C gerührt. Nach langsamer Erwämung auf Raumtemperatur wird die Reaktionsmischung mit Wasser hydrolisiert und dann auf Ether gegossen, die wässerige Phase mit Ether extrahiert und sodann die organische Phase mit Magnesiumsulfat getrocknet. Das Lösungsmittel wird am Rotationsverdampfer abdestilliert und der Rückstand an Kieselgel mit Petrolether/Essigsäureethylester (9:1) gereinigt.

### C. Synthese von 2,5-Diamino-1-aminomethylbenzolen

0,033 g (0,0001 mol) N-(4-tert.Butyloxycarbonylamino-2-formyl-phenyl) carbaminsäure-tert.butylester aus Stufe **B** und 0,00015 mol des entsprechenden Amins werden in 1,2-Dichlorethan gelöst. Anschliessend werden 0,1 ml einer Essigsäurelösung (1 M in 1,2-Dichlorethan) und 0.06 g NaBH(OAc)3 (0,0003 mol) zugegeben und die Reaktionmischung wird 5 bis 15 Stunden bei Raumtemperatur gerührt.

Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt.
Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.
**a1**.2-Ethylaminomethyl-1,4-diamino-benzol-Hydrochlorid
   Verwendetes Amin: Ethylamin
   Ausbeute: 0,025 g (91 % der Theorie)
   Masspektrum: MH⁺166 (100)
**b1**.2-(Isopropylamino-methyl)-1,4-diamino-benzol-Hydrochlorid
   Verwendetes Amin: Isopropylamin
   Ausbeute: 0,017 g (59 % der Theorie)
   Masspektrum: MH⁺180 (100)
**c1**.2-Propylaminomethyl-1,4-diamino-benzol-Hydrochlorid
   Verwendetes Amin: Propylamin
   Ausbeute: 0,025 g (87 % der Theorie)
   Masspektrum: MH⁺180 (100)
**d1**. 2-Pyrrolidin-1-ylmethyl-1,4-diamino-benzol-Hydrochlorid
   Verwendetes Amin: Pyrrolidin
   Ausbeute: 0,025 g (83 % der Theorie)
   Masspektrum: MH⁺ 192 (100)

**e1**. 2-[(2-Methoxy-ethylamino)-methyl]-1,4-diamino-benzol-Hydrochlorid
   Verwendetes Amin: 2-Methoxy-ethylamin
   Ausbeute: 0,025 g (82 % der Theorie)
   Masspektrum: MH⁺ 196 (100)

**f1**. 2-Morpholin-4-ylmethyl-1,4-diamino-benzol-Hydrochlorid
   Verwendetes Amin: Morpholin
   Ausbeute: 0,025 g (79 % der Theorie)
   Masspektrum: MH⁺ 208 (100)
**g1**. 2-(2,5-Diamino-benzylamino)-butan-1-ol-Hydrochlorid
   Verwendetes Amin: 2-Amino-1-butanol
   Ausbeute: 0,025 g (78 % der Theorie)
   Masspektrum: MH⁺ 210 (100)

**h1**. 2-{[(Furan-2-ylmethyl)-amino]-methyl}-1,4-diamino-benzol-Hydrochlorid
   Verwendetes Amin: Furfurylamin
   Ausbeute: 0,025 g (76 % der Theorie)
   Masspektrum: MH⁺ 218 (100)
**i1**. N-(2,5-Diamino-benzyl)-O,N-dimethyl-hydroxylamine-Hydrochlorid
   Verwendetes Amin: O,N-dimethyl-hydroxylamin
   Ausbeute: 0,025 g (86 % der Theorie)
   Masspektrum: MH⁺ 182 (100)
**j1**. 2-(4-Methyl-piperazin-1-ylmethyl)-1,4-diamino-benzol-Hydrochlorid
   Verwendetes Amin: 4-Methyl-piperazin
   Ausbeute: 0,025 g (68 % der Theorie)
   Masspektrum: MH⁺ 221 (100)
**k1**. 1-(2,5-Diamino-benzyl)-piperidin-4-ol-Hydrochlorid
   Verwendetes Amin: 4-Hydroxy-piperidin
   Ausbeute: 0,025 g (76 % der Theorie)
   Masspektrum: MH⁺ 222 (100)

**l1**. N-[2-(2,5-Diamino-benzylamino)-ethyl]-acetamid-Hydrochlorid N-Acetyl-ethylendiamin
   Verwendetes Amin: Ethylamin
   Ausbeute: 0,025 g (75 % der Theorie)
   Masspektrum: MH⁺ 223 (100)
**m1**. 2-[(2-Morpholin-4-yl-ethylamino)-methyl]-1,4-diamino-benzol-Hydrochlorid
   Verwendetes Amin: 4-(2-ethylamino)-morpholin
   Ausbeute: 0,025 g (63 % der Theorie)
   Masspektrum: MH⁺ 251 (100)
**n1**. 2-Allylaminomethyl-1,4-diamino-benzol-Hydrochlorid
   Verwendetes Amin: Allylamin
   Ausbeute: 0,025 g (87 % der Theorie)
   Masspektrum: MH⁺ 178 (100)
**o1**. 2-(2,5-Diamino-benzylamino)-propan-1-ol-Hydrochlorid
   Verwendetes Amin: 2-Amino-propanol
   Ausbeute: 0,025 g (82 % der Theorie)
   Masspektrum: MH⁺ 196 (100)
**p1**. 2-[(3-Imidazol-1-yl-propylamino)-methyl]-1,4-diamino-benzol-Hydrochlorid
   Verwendetes Amin: 1-(3-aminopropyl)-imidazol
   Ausbeute: 0,025 g (64 % der Theorie)
   Masspektrum: MH⁺ 246 (100)
**q1**. 2-{[(Tetrahydro-furan-2-ylmethyl)-amino]-methyl}-1,4-diamino-benzol-Hydrochlorid
   Verwendetes Amin: Tetrahydrofurfurylamin
   Ausbeute: 0,025 g (76 % der Theorie)
   Masspektrum: MH⁺ 222 (100)
**r1**. 4-(2,5-Diamino-benzylamino)-anilin-Hydrochloride
   Verwendetes Amin: 4-tert.-Butyloxycarbonylamino-anilin
   Ausbeute: 0,025 g (67 % der Theorie)
   Masspektrum: MH⁺ 229 (100)
**s1**. 3-(2,5-Diamino-benzylamino)-phenol-Hydrochloride
   Verwendetes Amin: 3-Aminophenol
   Ausbeute: 0,025 g (74 % der Theorie)
   Masspektrum: MH⁺ 230 (100)
**t1**. 5-(2,5-Diamino-benzylamino)-2-methyl-phenol-Hydrochlorid
   Verwendetes Amin: 3-Amino-6-methyl-phenol
   Ausbeute: 0,025 g (71 % der Theorie)
   Masspektrum: MH⁺ 244 (100)
**u1.** 2-[(2-Dimethylamino-ethylamino)-methyl]-1,4-diamino-benzol-Hydrochlorid
   Verwendetes Amin: 2-Dimethylamino-ethylamin
   Ausbeute: 0,016 g (45 % der Theorie)
   Masspektrum: MH⁺ 209 (100)
**v1.** 4-(2,5-Diamino-benzylamino)-butan-1-ol-Hydrochlorid
   Verwendetes Amin: 4-Amino-butanol
   Ausbeute: 0,022 g (69 % der Theorie)
   Masspektrum: MH⁺ 210 (100)
**w1.** 2-[(3-Ethoxy-propylamino)-methyl]-1,4-diamino-benzol-Hydrochlorid
   Verwendetes Amin: 3-Ethoxy-propylamin
   Ausbeute: 0,025 g (75 % der Theorie)
   Masspektrum: MH⁺ 224 (100)
**x1**. 2-[(3-Methoxy-phenylamino)-methyl]-1,4-diamino-benzol-Hydrochlorid
   Verwendetes Amin: 3-Methoxy-anilin
   Ausbeute: 0,025 g (71 % der Theorie)
   Masspektrum: MH⁺ 244 (100)
**y1**. 2-f(4-Chloro-phenylamino)-methyl]- 1,4-diamino-benzol-Hydrochlorid
   Verwendetes Amin: 4-Chlor-anilin
   Ausbeute: 0,025 g (70 % der Theorie)
   Masspektrum: M⁺ 248 (100)
**z1**.2-[(Cyclopropylmethyl-amino)-methyl]-1,4-diamino-benzol-Hydrochlorid
   Verwendetes Amin: Aminomethyl-cyclopropan
   Ausbeute: 0,017 g (56 % der Theorie)
   Masspektrum: MH⁺ 192 (100)
**a2.**2-(2,5-Diamino-benzylamino)-4-nitro-phenol-Hydrochlorid
   Verwendetes Amin: 2-Amino-4-nitro-phenol
   Ausbeute: 0,025 g (65 % der Theorie)
   Masspektrum: MH⁺ 275 (100)
**b2**.2-[(4-Chlor-benzylamino)-methyl-1,4-diamino-benzol-Hydrochlorid
   Verwendetes Amin: 4-Chlor-benzylamin
   Ausbeute: 0,025 g (67 % der Theorie)
   Masspektrum: M⁺ 262 (100)
**c2**.2-[(2,5-Diamino-benzyl)-methyl-aminol-ethanol-Hydrochlorid
   Verwendetes Amin: 2-Methylamino-ethanol
   Ausbeute: 0,025 g (82 % der Theorie)
   Masspektrum: MH⁺ 196 (100)
**d2**.2-[(2,5-Diamino-benzyl)-ethyl-amino]-ethanol-Hydrochlorid
   Verwendetes Amin: 2-Ethylamino-ethanol
   Ausbeute: 0,025 g (78 % der Theorie)
   Masspektrum: MH⁺ 210 (100)
**e2**.2-{[(Pyridin-4-ylmethyl)-amino]-methyl}-1,4-diamino-benzol-Hydrochlorid
   Verwendetes Amin: 4-Picolylamin
   Ausbeute: 0,025 g (67 % der Theorie)
   Masspektrum: MH⁺ 229 (100)
**f2**. 1-[3-(2,5-Diamino-benzylamino)-propyl]-pyrrolidin-2-on-Hydrochlorid
   Verwendetes Amin: 1-(3-aminopropyl)-2-pyrrolidon
   Ausbeute: 0,025 g (67 % der Theorie)
   Masspektrum: MH⁺ 263 (100)
**g2**.2-(4-Amino-2-methyl-phenyl)aminomethyl-1,4-diamino-benzol-Hydrochlorid und 2-(4-Amino-3-methyl-phenyl)aminomethyl-1,4-diamino-benzol-Hydrochlorid
   Verwendetes Amin: 4-tert.-Butyloxycarbonylamino-3-methyl-anilin und 4-tert.-Butyloxycarbonylamino-2-methyl-anilin
   Ausbeute: 0,021 g (27 % der Theorie)
   Masspektrum: MH⁺ 243 (80)
**h2**.2-[5-Amino-2-(2,5-diamino-benzylamino)-phenyl]-ethanol-Hydrochloride und 2-[2-Amino-5-(2,5-diamino-benzylamino)-phenyl]-ethanol-Hydrochlorid
   Verwendetes Amin: 4-tert.-Butyloxycarbonylamino-3-(2-hydroxyethyl) anilin und 4-tert.-Butyloxycarbonylamino-2-(2-hydroxyethyl)-anilin
   Ausbeute: 0,025 g (30 % der Theorie)
   Masspektrum: MH⁺ 273 (100)
**i2.** 2-(3-Amino-phenyl)aminomethyl-1,4-diamino-benzol-Hydrochlorid
   Verwendetes Amin: 3-tert.-Butyloxycarbonylamino-anilin
   Ausbeute: 0,025 g (67 % der Theorie)
   Masspektrum: MH⁺ 229 (100)
**j2.** 4-[2-(2,5-Diamino-benzylamino)-ethyl]-benzenesulfonamid-Hydrochlorid
   Verwendetes Amin: 4-(2-Aminoethyl)-benzensulfonamid
   Ausbeute: 0,025 g (58 % der Theorie)
   Masspektrum: MH⁺ 321 (100)
**k2**.2-[4-Amino-2-(2,5-diamino-benzylamino)-phenoxy]-ethanol-Hydrochlorid
   Verwendetes Amin: 4-tert.-Butyloxycarbonylamino-2-amino-(2-hydroxy)-ethoxy-benzol
   Ausbeute: 0,025 g (58 % der Theorie)
   Masspektrum: MH⁺ 289 (100)
**l2**. 2-[(2,5-Diamino-benzyl)-(2-hydroxy-ethyl)-amino]-ethanol-Hydrochlorid
   Verwendetes Amin: Diethanolamin
   Ausbeute: 0,025 g (75 % der Theorie)
   Masspektrum: MH⁺ 226 (100)
**m2.** [1-(2,5-Diamino-benzyl)-pyrrolidin-2-yl]-methanol-Hydrochlorid
   Verwendetes Amin: Prolinol
   Ausbeute: 0,025 g (76 % der Theorie)
   Masspektrum: MH⁺ 222 (100)
**n2.** 1-(2,5-Diamino-benzyl)-pyrrolidin-3-ol-Hydrochlorid
   Verwendetes Amin: 3-Hydroxy-pyrrolidin
   Ausbeute: 0,025 g (79 % der Theorie)
   Masspektrum: MH⁺ 208 (100)
**o2.1**-(2,5-Diamino-benzyl)-pyrrolidin-2-carbonsäureamid-Hydrochlorid
   Verwendetes Amin: Prolinamid
   Ausbeute: 0,025 g (73 % der Theorie)
   Masspektrum: MH⁺ 235 (100)
**p2.1**-(2,5-Diamino-benzyl)-piperidin-3-ol-Hydrochlorid
   Verwendetes Amin: 3-hydroxypiperidin
   Ausbeute: 0,025 g (76 % der Theorie)
   Masspektrum: MH⁺ 222 (100)
**q2**.2-(2,5-Diamino-benzylamino)-propan-1,3-diol-Hydrochlorid
   Verwendetes Amin: 3-Amino-1,2-propandiol
   Ausbeute: 0,015 g (47 % der Theorie)
   Masspektrum: MH⁺ 212 (100)
**r2**. 2-(2,5-Diamino-benzylamino)-3-hydroxy-propionamid-Hydrochlorid
   Verwendetes Amin: 3-Hydory-2-amino-propionamid
   Ausbeute: 0,025 g (75 % der Theorie)
   Masspektrum: MH⁺ 225 (100)
**s2**.2-(2,5-Diamino-benzylamino)-bernsteinsäure-Hydrochlorid
   Verwendetes Amin: Asparagin
   Ausbeute: 0,037 g (102 % der Theorie)
   Masspektrum: MH⁺ 253 (100)
**t2**. 2-Cyclopropylaminomethyl-1,4-diamino-benzol-Hydrochlorid
   Verwendetes Amin: Cyclopropylamin
   Ausbeute: 0,025 g (87 % der Theorie)
   Masspektrum: MH⁺ 178 (100)
**u2.**2-(2,5-Diamino-benzylamino)-ethanol-Hydrochlorid
   Verwendetes Amin: Ethanolamin
   Ausbeute: 0,025 g (86 % der Theorie)
   Masspektrum: MH⁺ 182 (100)
**v2**. (2,5-Diamino-benzylamino)-essigsäure-Hydrochlorid
   Verwendetes Amin: Glycin
   Ausbeute: 0,025 g (82 % der Theorie)
**w2.** 4-(2,5-Diamino-benzylamino)-phenol-Hydrochlorid
   Verwendetes Amin: 4-Aminophenol
   Ausbeute: 0,025 g (74 % der Theorie)
   Masspektrum: MH⁺ 230 (100)
**x2**.2-(Benzo[1,3]dioxol-5-ylaminomethyl)-1,4-diamino-benzol-Hydrochlorid
   Verwendetes Amin: 3,4-Methylendioxy-anilin
   Ausbeute: 0,025 g (68 % der Theorie)
   Masspektrum: MH⁺ 258 (100)
**y2**.[(2,5-Diamino-benzyl)-methyl-amino]acetonitril-Hydrochlorid
   Verwendetes Amin: Methylaminoacetonitril
   Ausbeute: 0,025 g (83 % der Theorie)
   Masspektrum: MH⁺ 191 (100)
**z2**. 2-Pentylaminomethyl-benzene-1,4-diamin-Hydrochlorid
   Verwendetes Amin: Pentylamin
   Ausbeute: 0,025 g (79 % der Theorie)
   Masspektrum: MH⁺ 208 (100)
**a3.**2-[(3-Dimethylamino-propylamino)-methyl]-1,4-diamino-benzol-Hydrochlorid
   Verwendetes Amin: 3-Dimethylamino-propylamin
   Ausbeute: 0,025 g (68 % der Theorie)
   Masspektrum: MH⁺ 223 (100)
**b3.**2-{[2-(5-Nitro-pyridin-2-ylamino)-ethylamino]-methyl}-1,4-diamino-benzol-Hydrochlorid
   Verwendetes Amin: 2-Amino-5-nitro-pyridin
   Ausbeute: 0,025 g (56 % der Theorie)
   Masspektrum: MH⁺ 303 (100)
**c3.**2-[(2-Amino-ethylamino)-methyl]- ,4-diamino-benzol-Hydrochlorid
   Verwendetes Amin: Ethylendiamin
   Ausbeute: 0,025 g (77 % der Theorie)
   Masspektrum: MH⁺ 181 (100)
**d3.**3-[2-(2,5-Diamino-benzylamino)-1-hydroxy-ethyl]-phenol-Hydrochloride
   Verwendetes Amin: 1-(3-Hydroxyphenyl)-2-aminoethanol
   Ausbeute: 0,025 g (65 % der Theorie)
   Masspektrum: MH⁺274 (100)
**e3**.2-[(4-Methyl-pyridin-2-ylamino)-methyl]-1,4-diamino-benzol-Hydrochlorid
   Verwendetes Amin: 2-Picolylamin
   Ausbeute: 0,022 g (65 % der Theorie)
   Masspektrum: MH⁺ 229 (100)
**f3.** 2-(2,5-Diamino-benzyl)-1-methyl-1,2,3,4-tetrahydro-isochinolin-6,7-diol-Hydrochlorid
   Verwendetes Amin: 1-Methyl-6,7-dihydroxy-1,2,3,4-tetrahydroisochinolin Ausbeute: 0,015 g (37 % der Theorie)
   Masspektrum: MH⁺ 300 (100)
**g3**.2-(2.5-Diamino-benzylamino)-4-methylsulfanyl-buttersäure-Hydrochlorid
   Verwendetes Amin: 2-Amino-4-methylmercapto-buttersäure
   Ausbeute: 0,012 g (32 % der Theorie)
**h3**.1-(2,5-Diamino-benzyl)-pyrrolidine-2-carbonsäure-Hydrochlorid
   Verwendetes Amin: Pyrrolidin-2-carbonsäure
   Ausbeute: 0,025 g (72 % der Theorie)
   Masspektrum: MH⁺ 236 (55)
**i3**. 2-Phenylaminomethyl-1,4-diamino-benzol-Hydrochlorid
   Verwendetes Amin: Anilin
   Ausbeute: 0,025 g (77 % der Theorie)
   Masspektrum: MH⁺ 214 (100)
**j3**. 2-(4-Dimethylamino-phenylaminomethyl-1,4-diamino-benzol-Hydrochlorid
   Verwendetes Amin: 4-Amino-N,N-dimethylanilin
   Ausbeute: 0,025 g (62 % der Theorie)
   Masspektrum: MH⁺ 257 (100)
**k3.** 1-[3-(2,5-Diamino-benzylamino)-phenyl]-ethanol-Hydrochloride
   Verwendetes Amin: 3-(1-hydroxyethyl)-anilin
   Ausbeute: 0,025 g (68 % der Theorie)
   Masspektrum: MH⁺ 258 (100)
**l3.** 2-[(3,4-Dimethoxy-phenylamino)-methyl]-1,4-diamino-benzol-Hydrochlorid
   Verwendetes Amin: 3,4-Dimethoxy-anilin
   Ausbeute: 0,025 g (65 % der Theorie)
   Masspektrum: MH⁺ 274 (100)
**m3.** 2-[(3-Fluoro-2-methoxy-phenylamino)-methyl]-1,4-diamino-benzol-Hydrochlorid
   Verwendetes Amin: 3-Fluor-2-methoxy-anilin
   Ausbeute: 0,021 g (57 % der Theorie)
   Masspektrum: MH⁺ 262 (100)
**n3**.4-Chloro-2-(2,5-diamino-benzylamino)-phenol-Hydrochloride
   Verwendetes Amin: 4-Chlor-2-amino-phenol
   Ausbeute: 0,025 g (67 % der Theorie)
   Masspektrum: MH⁺ 264 (100)
**o3**.2-[(4-Trifluoromethyl-phenylamino)-methyl]- 1,4-diamino-benzol-Hydrochlorid
   Verwendetes Amin: 4-Trifluormethyl-anilin
   Ausbeute: 0,025 g (64 % der Theorie)
   Masspektrum: MH⁺ 282 (100)
**p3**.2-(p-Tofylamino-methyl)- 1,4-diamino-benzol-Hydrochlorid
   Verwendetes Amin: 4-Methyl-anilin
   Ausbeute: 0,025 g (74 % der Theorie)
   Masspektrum: MH⁺ 228 (100)

### Beispiele 2: Synthese von 2,5-Diamino-1-(1-amino-ethyl)-benzol-Derivaten

### A. Synthese von (4-tert.-Butoxycarbonylamino-3-(1-hydroxy-ethylphenyl)-carbaminsäure-tert-buylester

3,3 g (0,01 mol) (4-tert.-Butoxycarbonylamino-3-brom-phenyl) carbaminsäure-tert.-butylester werden unter Argon in 200 ml Diethylether gelöst. Dann werden bei -25 °C zunächst 20 ml einer 1,6molaren Methyllithium-Lösung und sodann 16 ml einer 1,6molaren tert.-Butyllithium-Lösung zugegeben. Nach einer Stunde werden 1,2 ml (0,02 mol) Acetaldehyd zugegeben und die Reaktionsmischung langsam auf 20 °C erwärmt. Nach Beendigung der Reaktion wird die Reaktionsmischung mit Wasser hydrolisiert, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (8:2) gereinigt.
Es werden 3,0 g (85% der Theorie) (4-tert.-Butoxycarbonylamino-3-hydroxymethyl-phenyl)-carbaminsäure-tert.-butylester mit einem Schmelzpunkt von 189 °C erhalten.

### B. Synthese von (4-tert.-Butoxycarbonylamino-3-(1-amino-ethyl-phenyl)-carbaminsäure-tert.-butylester

3,5 g (4-tert-Butoxycarbonylamino-3-(1-hydroxy-ethyl-phenyl)-carbaminsäure-tert.-butylester (0,01 mol) aus Stufe **A** werden in 30 ml Dichlormethan gelöst. Dann werden bei 4 °C 1,3 g (0,013 mol) Triethylamin und 2,4 g (0,01 mol) Mesitylensulfochlorid zugegeben. Die Lösung wird zunächst eine Stunde bei 4 °C und anschließend eine Stunde bei Raumtemperatur gerührt. Das Lösungsmittel wird am Rotationsverdampfer abdestilliert und der Rückstand an Kieselgel mit Hexan/Essigsäureethylester (1:5) gereinigt.
Anschliessend wird das Produkt in 30 ml Dimethylsulfoxid gelöst und mit 3,5 g (0,05 mol) Natriumazid versetzt und sodann die Reaktionsmischung auf 60 °C erwärmt. Nach Beendigung der Reaktion wird die Reaktionsmischung in Essigsäureethylester/Wasser gegossen und die organische Phase mit Magnesiumsulfat getrocknet. Das Lösungsmittel wird am Rotationsverdampfer abdestilliert und der Rückstand an Kieselgel mit Essigsäureethylester/Hexan (1:6) gereinigt.
Das so erhaltene Produkt wird in Ethanol gelöst und unter Zusatz von 200 mg eines Palladium-Aktivkhole-Katalysators (10%ig) und 1,8 g (0,03mol) Essigsäure bei 25 °C hydriert. Nach 4 Stunden wird der Katalysator abfiltriert. Das Lösungsmittel wird am Rotationsverdampfer abdestilliert und der Rückstand an Kieselgel mit Chloroform/Methanol/Triethylamin (50:10:1) gereinigt.

Es werden 1,0 g (28% der Theorie) (4-tert.-Butoxycarbonylamino-3-aminomethyl-phenyl)-carbaminsäure-tert.-butylester mit einem Schmelzpunkt von 170 °C erhalten.

### C. Synthese von 1,4-Diamino-2-(1-amino-ethyl)-benzolen

0,033 g (0,0001 mol) (4-tert-Butoxycarbonylamino-3-(1-amino-ethylphenyl)-carbaminsäure-tert.-butylester aus Stufe **B** und 0,00015 mol des entsprechenden Aldehyds werden in 1,2-Dichlorethan gelöst. Anschliessend werden 0,1 ml einer Essigsäurelösung (1 M in 1,2-Dichlorethan) und
0,06 g (0,0003 mol) NaBH(OAc)3 hinzugegeben und die Reaktionsmischung 5 bis 15 Stunden bei Raumtemperatur gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt.
Anschliessend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.
a**.** 1,4-Diamino-2-(1-butylamino-ethyl)-benzol-Hydrochlorid
   Verwendeter Aldehyd: Butyraldehyd
   Ausbeute: 0,025 g (78 % der Theorie)
   Masspektrum: MH+ 208(100)
**b**. 1,4-Diamino-2-[1-(3-methyl-butylamino)-ethyl]-benzol-Hydrochlorid
   Verwendeter Aldehyd: 3-Methyl-butylraldehyd
   Ausbeute: 0,025 g (75 % der Theorie)
   Masspektrum: MH+ 222(100)
**c.** 1,4-Diamino-2-(1-benzylamino-ethyl)-benzol-Hydrochlorid
   Verwendeter Aldehyd: Benzaldehyd
   Ausbeute: 0,025g (71 % der Theorie)
   Masspektrum: MH+ 242(100)
**d**. 1,4-Diamino-2-{1-[(pyridin-2-ylmethyl)-amino]-ethyl}-benzol-Hydrochlorid
   Verwendeter Aldehyd: Pyridin-2-carbaldehyd
   Ausbeute: 0,025g (64 % der Theorie)
   Masspektrum: MH+ 243(20)
**e.** 1,4-Diamino-2-{1-[(pyridin-3-ylmethyl)-amino]-ethyl}-benzol-Hydrochlorid
   Verwendeter Aldehyd: Pyridin-3-carbaldehyd
   Ausbeute: 0,025g (64 % der Theorie)
   Masspektrum: MH+ 243(50)
**f.** 1,4-Diamino-2-{1-[(pyridin-4-ylmethyl)-amino]-ethyl}-benzol-Hydrochlorid
   Verwendeter Aldehyd: Pyridin-4-carbaldehyd
   Ausbeute: 0,025g (64 % der Theorie)
   Masspektrum: MH+ 243(100)
**g**. 1,4-Diamino-2-{1-[(thiophen-2-ylmethyl)-amino]-ethyl}-benzol-Hydrochlorid
   Verwendeter Aldehyd: Thiophen-2-carbaldehyd
   Ausbeute: 0,025g (70 % der Theorie)
   Masspektrum: MH+ 248(100)
**h**. 1,4-Diamino-2-{1-[(thiophen-3-ylmethyl)-amino]-ethyl}-benzol-Hydrochlorid
   Verwendeter Aldehyd: Thiophen-2-carbaldehyd
   Ausbeute: 0,025g (70 % der Theorie)
   Masspektrum: MH+ 248(100)
**i.** 1,4-Diamino-2-[1-(Cyclohexylmethyl-amino)-ethyl]-benzol-Hydrochlorid
   Verwendeter Aldehyd: Cyclohexancarbaldehyd
   Ausbeute: 0,025g (70 % der Theorie)
   Masspektrum: MH+ 248(100)
**j.** 4-{[1-(2,5-Diamino-Phenyl)-ethylaminol-methyl}-Phenol-Hydrochlorid
   Verwendeter Aldehyd: 4-Hydroxy-benzaldehyd
   Ausbeute: 0,025g (68 % der Theorie)
   Masspektrum: MH+ 258(100)
**k.** 1,4-Diamino-2-[1-(4-dimethylamino-benzylamino)-ethyl]-benzol-Hydrochlorid
   Verwendeter Aldehyd: 4-Dimethylamino-benzaldehyd
   Ausbeute: 0,020g (46 % der Theorie)
   Masspektrum: MH+ 285(100)
**l.** 1,4-Diamino-2-[1-(4-nitro-benzylamino)-ethyl]-benzol-Hydrochlorid
   Verwendeter Aldehyd: 4-Nitrobenzaldehyd
   Ausbeute: 0,025g (63 % der Theorie)
   Masspektrum: MH+ 286(100)
**m.** 2-{[1-(2,5-Diamino-phenyl)-ethylamino]-methyl}-4-nitro-phenol-Hydrochlorid
   Verwendeter Aldehyd: 2-Hydroxy-5-nitro-benzaldehyd
   Ausbeute: 0,025g (60 % der Theorie)
   Masspektrum: MH+ 303(100)
**n**. 1,4-Diamino-2-[1-(4-pyrrolidin-1-yl-benzylamino)-ethyl]-benzol-Hydrochlorid
   Verwendeter Aldehyd: 4-Pyrrolidino-benzaldehyd
   Ausbeute: 0,025g (54 % der Theorie)
   Masspektrum: MH+ 311(20)
**p.** 1,4-Diamino-2-{1-[(benzo[1,3]dioxol-5-ylmethyl)-amino]-ethyl}benzol-Hydrochlorid
   Verwendeter Aldehyd: 3,4-Methylendioxy-benzaldehyd
   Ausbeute: 0,025g (63 % der Theorie)
   Masspektrum: MH+ 286(100)
**q.** 1,4-Diamino-2-[1-(3-chlor-benzylamino)-ethyl]-benzol-Hydrochlorid
   Verwendeter Aldehyd: 3-Chlor-benzaldehyd
   Ausbeute: 0,025g (64 % der Theorie)
   Masspektrum: MH+ 276(100)

### D. Synthese von 1,4-Diamino-2-(1-amino-ethyl)-benzolen

0,033 g (0,0001 mol) (4-tert-Butoxycarbonylamino-3-(1-amino-ethylphenyl)-carbaminsäure-tert.-butylester aus Stufe **B** werden in 25 ml Ethanol gelöst. Anschließend werden unter Rückfluß 0,00015 mol des entsprechenden Fluorderivats zugegeben. Nach Beendigung der Reaktion wird die Reaktionsmischung in Wasser gegossen, die wässerige Phase mit Essigsäureethylester extrahiert und sodann mit Magnesiumsulfat getrocknet. Das Lösungsmittel wird am Rotationsverdampfer abdestilliert und der Rückstand an Kieselgel mit Hexan/ Essigsäureethylester (5:1) gereinigt. Das so erhaltene Produkt wird in 4 ml Ethanol auf 50 °C erwärmt. Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.
**r.** 1,4-Diamino-2-[1-2-nitro-phenlamino)-ethyl]-benzol-Hydrochlorid
   Verwendetes Fluorderivat: 1-Fluor-2-nitro-benzol
   Ausbeute: 0,025g (65 % der Theorie)
   Masspektrum: MH+ 273(100)
**s.** 1,4-Diamino-2-[1-(4-fluor-2-nitro-phenylamino)-ethyl-benzol-Hydrochlorid
   Verwendetes Fluorderivat: 1,4-Difluor-3-nitro-benzol
   Ausbeute: 0,020g (50 % der Theorie)
   Masspektrum: MH+ 291(100)
**t.** 1,4-Diamino-2-[1-(5-fluor-2-nitro-phenylamino)-ethyl]-benzol-Hydrochlorid
   Verwendetes Fluorderivat: 1,5-Difluor-2-nitro-benzol
   Ausbeute: 0,025g (62 % der Theorie)
**u.** 1,4-Diamino-2-[1-(2-Fuor-6-nitro-phenylamino)-ethyl]-benzol-Hydrochlorid
   Verwendetes Fluorderivat: 1,2-Fluor-6-nitro-benzol
   Ausbeute: 0,025g (62 % der Theorie)
   Masspektrum: MH+ 291(100)
**v**. 2-[1-(2,5-Diamino-phenyl)-ethylamino]-5-nitro-benzoesäure-Hydrochlorid
   Verwendetes Fluorderivat: 2-Fluor-5-nitro-benzoesäure
   Ausbeute: 0,025g (64 % der Theorie)
   Masspektrum: MH+ 317(100)
**w**. 1,4-Diamino-2-[1-(4-bromo-2-nitro-Dhenylamino)-ethyl]-benzol-Hydrochlorid
   Verwendetes Fluorderivat: 1-Brom-4-fluor-3-nitro-benzol Ausbeute: 0,018g (39 % der Theorie)
**x.** 1,4-Diamino-2-[1-(4-Amino-2-nitro-Dhenylamino)-ethyl]-benzol-Hydrochlorid
   Verwendetes Fluorderivat: 1-Fluor-2-nitro-4-amino-benzol Ausbeute: 0,016g (36 % der Theorie)
   Masspektrum: MH+ 288(80)

### Beispiele 3 bis 70: Haarfärbemittel

Es werden Haarfärbelösungen der folgenden Zusammensetzung hergestellt:

| | |
|---|---|
| 0,0125 mmol | Entwicklersubstanz der Formel (I) gemäss Tabelle 2 |
| 0,0125 mmol | Kupplersubstanz gemäß Tabelle 2 |
| 0,01g | Kaliumoleat (8prozentige wässrige Lösung) |
| 0,01g | Ammoniak (22prozentige wässrige Lösung) |
| 0,01g | Ethanol |
| 0,003 g | Ascorbinsäure |
| ad 1,0 g | Wasser |

1 g der vorstehenden Färbelösung wird unmittelbar vor der Anwendung mit 1 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel** | **Entwicklersubstanz der Formel (I) aus Bsp. 1** | **Kupplersubstanz** | | | |
|---|---|---|---|---|---|
| | | **I. 1,3-Dihydroxybenzol** | **II. 1,3-Diamino-4-(2-hydroxy-ethoxy)-benzol*sulfat** | **III. 5-Amino-2-methylphenol** | **IV. 1-Naphtol** |
| **3.** | a1. | hell-lichtblond | graublau | mittelpurpur | graurosa |
| **4.** | b1. | hell-lichtblond | blau | hellpurpur | hell-graurosa |
| **5.** | c1. | hell-lichtblond | graublau | hellpurpur | graurosa |
| **6.** | d1. | hellblond | blau | mittelpurpur | hellpurpur |
| **7.** | e1. | hell-lichtblond | graublau | hellpurpur | graurosa |
| **8.** | f1. | hellblond | blau | mittelpurpur | violett |
| **9.** | g1. | hell-lichtblond | blau | mittelpurpur | graurosa |
| **10.** | h1. | hell-lichtblond | blau | mittelpurpur | graurosa |
| **11.** | i1. | hellblond | blau | mittelpurpur | violett |
| **12.** | j1. | hellblond | blau | hellpurpur | graurosa |
| **13.** | k1. | hellblond | blau | mittelpurpur | graurosa |
| **14.** | I1. | hell-lichtblond | blau | hellpurpur | graurosa |
| **15.** | m1. | hell-lichtblond | blau | hellpurpur | hellpurpur |
| **16.** | n1. | hell-lichtblond | blau | hellpurpur | graurosa |
| **17.** | o1. | hellblond | blau | mittelpurpur | hell-graurosa |
| **18.** | p1. | hell-lichtblond | blau | hellpurpur | graurosa |
| **19.** | q1. | hell-lichtblond | blau | hellpurpur | graurosa |
| **20.** | r1. | blau | tiefblau | blau | tiefblau |
| **21.** | s1. | rotbraun | graublau | graurosa | graurosa |
| **22.** | t1. | hellrosa | hell-graublau | hellrosa | graurosa |
| **23.** | u1. | hell-lichtblond | hell-graublau | hellpurpur | hell-graurosa |
| **24.** | v1. | hell-lichtblond | blau | hellpurpur | graurosa |
| **25.** | w1. | hell-lichtblond | blau | hellpurpur | graurosa |
| **26.** | x1. | hell-aschblond | blau | mittelpurpur | violett |
| **27.** | y1. | hellblond | blau | mittelpurpur | violett |
| **28.** | z1. | hell-lichtblond | graublau | hellpurpur | graurosa |
| **29.** | a2 | goldblond | grau | mittelpurpur | mittelpurpur |
| **30.** | b2 | hell-lichtblond | blau | hellpurpur | graurosa |
| **31.** | c2 | hellblond | blau | mittelpurpur | graurosa |
| **32.** | d2 | mittel-purpur | blau | mittelpurpur | violett |
| **33.** | e2 | hell-lichtblond | blau | mittelpurpur | mittelpurpur |
| **34.** | f2 | hell-lichtblond | blau | hellpurpur | graurosa |
| **35.** | g2 | violett | tiefblau | violett | blau |
| **36.** | h2 | violett | tiefblau | violett | violett |
| **37.** | i2 | tiefblau | tiefblau | graurosa | graublau |
| 38. | j2 | hell-lichtblond | graublau | mittelpurpur | graurosa |
| **39.** | k2 | graublau | graublau | graublau | graublau |
| **40.** | l2 | hellblond | blau | mittelpurpur | graurosa |
| **41.** | m2 | hellblond | blau | mittelpurpur | graurosa |
| **42.** | n2 | hellblond | blau | mittelpurpur | hell-graurosa |
| **43.** | o2 | hellblond | blau | mittelpurpur | graurosa |
| **44.** | p2 | hellblond | blau | mittelpurpur | graurosa |
| **45.** | q2 | hell-lichtblond | blau | mittelpurpur | hell-graurosa |
| **46.** | r2 | hellblond | tiefblau | purpur | violett |
| **47.** | s2 | hell-lichtblond | hell-graublau | hellpurpur | hellrosa |
| **48.** | t2 | hell-lichtblond | blau | mittelpurpur | graurosa |
| **49.** | u2 | hell-lichtblond | graublau | mittelpurpur | graurosa |
| **50.** | v2 | hell-lichtblond | graublau | hellpurpur | graurosa |
| **51.** | w2 | hellpurpur | hellgrau | hellpurpur | hellpurpur |
| **52.** | x2 | hell-aschblond | graublau | Not avialable | graurosa |
| **53.** | y2 | hellx-lichtblond | graublau | hellpurpur | hell-graurosa |
| **54.** | z2 | hell-lichtblond | graublau | hellpurpur | graurosa |
| **55.** | a3 | hell-lichtblond | blau | hellpurpur | hellpurpur |
| **56.** | b3 | hell-lichtblond | graublau | hellpurpur | graurosa |
| **57.** | c3 | hell-lichtblond | graublau | purpur | graurosa |
| **58.** | d3 | hell-lichtblond | graublau | mittelpurpur | graurosa |
| **59.** | e3 | hellblond | tiefblau | mittelpurpur | violett |
| **60.** | f3 | hell-lichtblond | hell-graublau | hellpurpur | graurosa |
| **61.** | g3 | hell-lichtblond | hell-graublau | hellrosa | hell-graurosa |
| **62.** | h3 | hell-lichtblond | graurosa | hellpurpur | hell-graurosa |
| **63.** | i3 | hellblond | tiefblau | mittelpurpur | violett |
| **64.** | j3 | hell-aschblond | graublau | purpur | graurosa |
| **65.** | k3 | hell-aschblond | blau | mittelpurpur | violett |
| **66.** | l3 | grau | blau | violett | graurosa |
| **67.** | m3 | hellblond | blau | mittelpurpur | violett |
| **68.** | n3 | hellblond | graublau | graupurpur | graurosa |
| **69.** | o3 | hellblond | blau | mittelpurpur | hellviolett |
| **70.** | p3 | hellblond | blau | mittelpurpur | violett |

### Beispiele 71 bis 80: Haarfärbemittel

Es werden Haarfärbelösungen der folgenden Zusammensetzung hergestellt:

| | |
|---|---|
| X g | Entwicklersubstanz E1 bis E1" der Formel (I) gemäss Tabelle 2 |
| U g | Entwicklersubstanz E2 bis E9 gemäss Tabelle 2 |
| Y g | Kupplersubstanz K11 bis K36 gemäss Tabelle 4 |
| Z g | direktziehender Farbstoff D1 bis D3 gemäss Tabelle 3 |
| 10,000 g | Kaliumoleat (8prozentige wässrige Lösung) |
| 10,000 g | Ammoniak (22prozentige wässrige Lösung) |
| 10,000 g | Ethanol |
| 0,300 g | Ascorbinsäure |
| ad 100,000 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die Färbeergebnisse sind in Tabelle 5 zusammengefasst.

### Beispiele 81 bis 86: Haarfärbemittel

Es werden cremeförmige Farbträgermassen der folgenden Zusammensetzung hergestellt:

| | |
|---|---|
| X g | Entwicklersubstanz E1 bis E1" der Formel (I) gemäss Tabelle 2 |
| Y g | Kupplersubstanz K11 bis K36 gemäss Tabelle 4 |
| Z g | direktziehender Farbstoff D2 gemäss Tabelle 3 |
| 15,0 g | Cetylalkohol |
| 0,3 g | Ascorbinsäure |
| 3,5 g | Natriumlaurylalkoholdiglycolethersulfat, 28%ige wässrige Lösung |
| 3,0 g | Ammoniak 22%ige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6prozentigen Wasserstoffperoxidlösung vermischt. Anschliessend wird das Gemisch auf das Haar aufgetragen. Nach einer Einwirkzeit von 30 Minuten wird das Haar mit Wasser gespült, mit einem handelsüblichen Shampoo gewaschen und getrocknet. Die Färbeergebnisse sind den nachfolgenden Tabellen 2 bis 6 zu entnehmen.

### Beispiele 87 bis 110: Haarfärbemittel

Es werden Haarfärbelösungen der folgenden Zusammensetzung hergestellt:

| | |
|---|---|
| 0,0125 mmol | Entwicklersubstanz der Formel (I) gemäss Tabelle 7 |
| 0,0125 mmol | Kupplersubstanz gemäß Tabelle 7 |
| 0,01g | Kaliumoleat (8prozentige wässrige Lösung) |
| 0,01g | Ammoniak (22prozentige wässrige Lösung) |
| 0,01g | Ethanol |
| 0,003 g | Ascorbinsäure |
| ad 1,0 g | Wasser |

1 g der vorstehenden Färbelösung wird unmittelbar vor der Anwendung mit 1 g einer 6prozentigen wässrigen Wasserstoffperoxidlösung vermischt. Anschliessend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 7 zusammengefaßt.

**Tabelle 2:**

| **Entwicklersubstanzen** | |
|---|---|
| **E1** | 2-Phenylaminomethyl-1,4-diamino-benzol-Hydrochlorid (gemäss Beispiel 1i3) |
| **E1'** | 2-[(4-Methyl-pyridin-2-ylamino)-methyl]-1,4-diamino-benzol-Hydrochlorid (gemäss Beispiel 1e3) |
| **E1"** | 2-[(2,5-Diamino-benzyl)-(2-hydroxy-ethyl)-amino]-ethanol; Hydrochlorid (gemäss Beispiel 1l2) |
| | |
| **E2** | 1,4-Diaminobenzol |
| **E3** | 2,5-Diamino-phenylethanol-sulfat |
| **E4** | 3-Methyl-4-amino-phenol |
| **E5** | 4-Amino-2-aminomethyl-phenol-dihydrochlorid |
| **E6** | 4-Amino-phenol |
| **E7** | N,N-Bis(2'-hydroxyethyl)-p-phenylendiamin-sulfat |
| **E8** | 4,5-Diamino-1-(2'-hydroxyethyl)-pyrazol-sulfat |
| **E9** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-Hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylharnstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1 ,3-dihydroxy-benzol |
| **K33** | 1-Chlor-2,4-dihydroxy-benzol |
| **K34** | 4-(2'-Hydroxyethyl)amino-1,2-methylendioxybenzol-hydrochlorid |
| **K35** | 3,4-Methylendioxy-phenol |
| **K36** | 2-Amino-5-methyl-phenol |

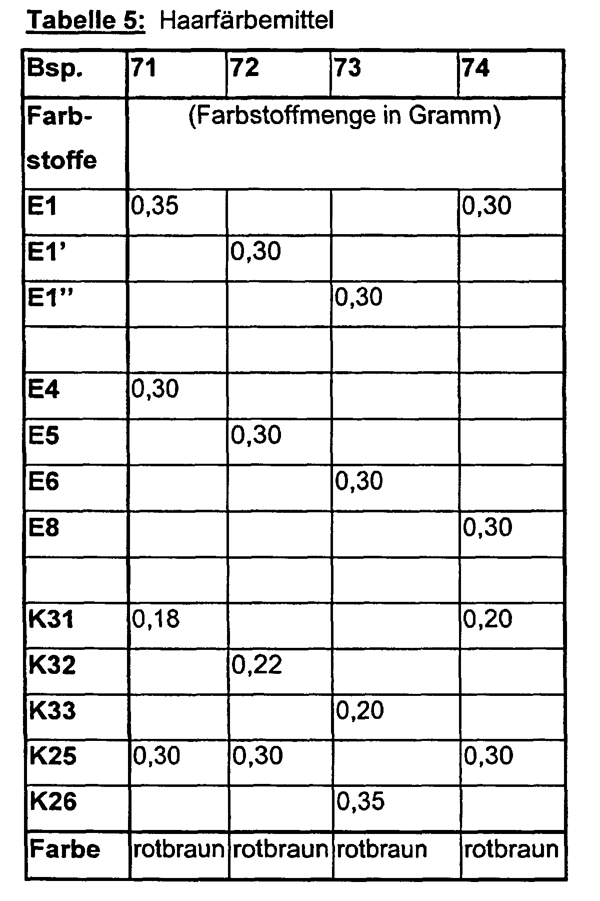

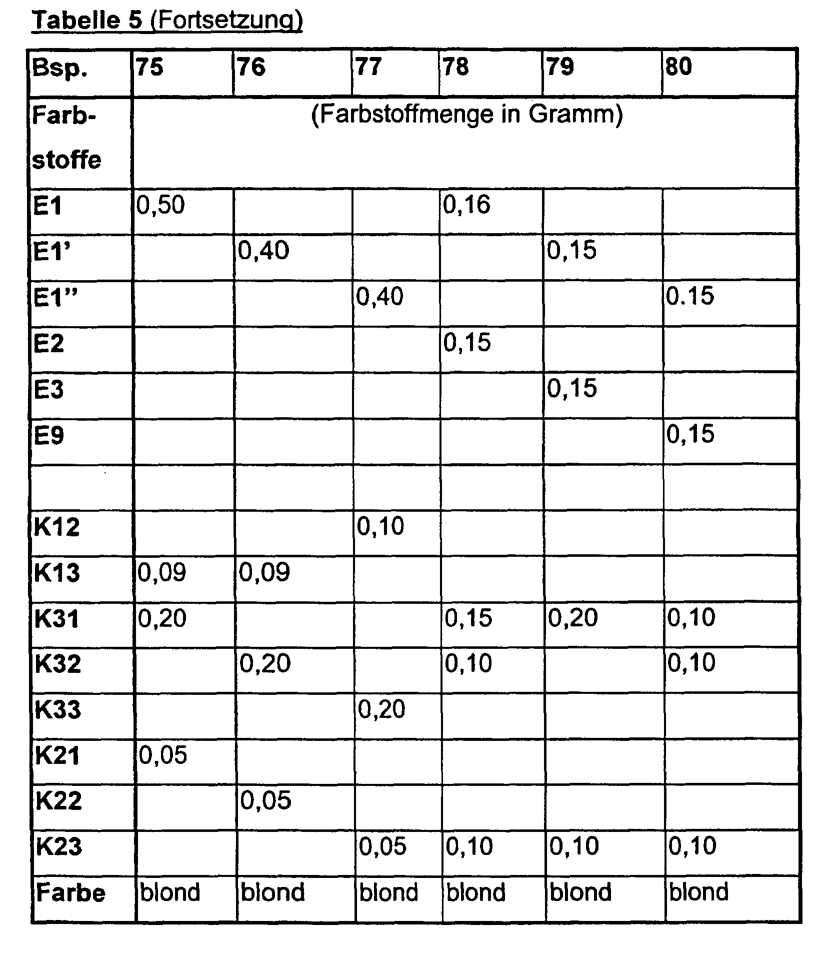

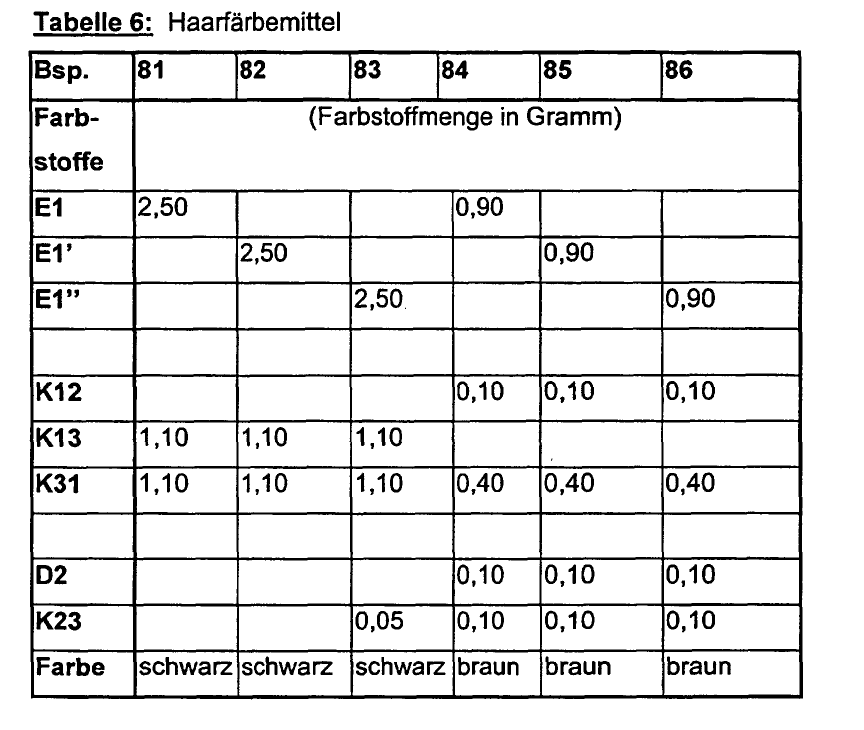

**Tabelle 7:**

| **Haarfärbemittel** | | | | | |
|---|---|---|---|---|---|
| **Beispiel** | **Entwickler-substanz der Formel (I) aus Bsp. 2** | Kupplersubstanz | | | |
| | | **I. 1,3-Dihydroxy-benzol** | **II. 1,3-Diamino-4-(2-hydroxy-ethoxy)-benzol*sulfat** | **III. 5-Amino-2-methylphenol** | **IV. 1-Naphtol** |
| **87.** | a. | hell-lichtblond | graublau | purpur | graurosa |
| **88.** | b. | hell-lichtblond | blau | hellpurpur | graurosa |
| **89.** | c. | hell-lichtblond | graublau | purpur | graurosa |
| **90.** | d. | hellblond | blau | mittelpurpur | violett |
| **91.** | e. | hell-lichtblond | graublau | hellpurpur | graurosa |
| **92.** | f. | hellblond | blau | mittelpurpur | grauviolett |
| 93. | g. | hell-lichtblond | graublau | hellpurpur | graurosa |
| **94.** | h. | hellblond | graublau | purpur | violett |
| **95.** | i. | hellblond | graublau | hellpurpur | Hellviolett |
| **96.** | j. | hellblond | blau | mittelpurpur | graurosa |
| **97.** | k. | hell-lichtblond | blau | hellpurpur | graurosa |
| **98.** | I. | hellblond | blau | purpur | Violett |
| **99.** | m. | gelb | braun | rotbraun | rotbraun |
| **100.** | n. | Hell-lichtblond | grau | hellpurpur | hellviolett |
| **101.** | o. | hellblond | blau | mittelpurpur | hell-graurosa |
| **102.** | p. | hell-lichtblond | graublau | hellpurpur | hellviolett |
| **103.** | q. | hell-lichtblond | graublau | hellpurpur | hellviolett |
| **104.** | r. | hellblond | grau | rotwein | grauschwarz |
| **105.** | s. | grün | schwarzblau | rotwein | grau |
| **106.** | t. | hellblond | graublau | hellpurpur | grau |
| **107.** | u. | hell-lichtblond | graublau | mittellpurpur | hell-graurosa |
| **108.** | v. | hell-lichtblond | graublau | rotbraun | grau |
| **109.** | w. | hell-lichtblond | graublau | mittellpurpur | grau |
| **110.** | x. | hellpurpur | rotblau | mittelpurpur | violett |

Alle in der vorliegenden Anmeldung enthaltenen Prozentangaben stellen soweit nicht anders angegeben Gewichtsprozente dar.

## Patentansprüche

1. 2-Aminoalkyl-1,4-diaminobenzol-Derivat der allgemeinen Formel (I) 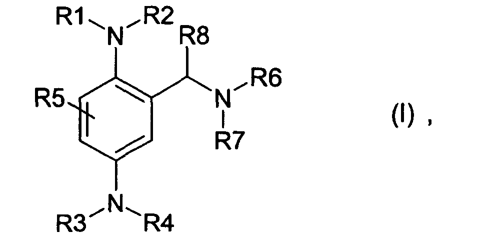 worin
**R1,R2,R3 und R4** unabhängig voneinander Wasserstoff, eine C₁C₆-Alkylgruppe, eine C₁-C₄-Hydroxyalkylgruppe, eine C₂-C₄ Dihydroxyalkylgruppe oder eine C₁-C₄-Alkoxy-(C₁-C₂)-alkylgruppe darstellen oder R1 und R2 einen viergliedrigen bis achtgliedrigen aliphatischen Ring bilden, wobei mindestens 2 der Reste R1 bis R4 Wasserstoff darstellen;
**R5** gleich Wasserstoff, einem Halogenatom, einer C₁-C₄-Alkylgruppe, einer C₁-C₄-Hydroxyalkylgruppe oder einer C₁-C₄-Alkoxygruppe ist;
**R6 und R7** unabhänging voneinander gleich Wasserstoff, einer C₁-C₂-Alkoxygruppe, einer C₁-C₆-Alkylgruppe, einer ungesättigten C₁-C₆-Alkylgruppe, einer C₁-C₄-Hydroxyalkylgruppe, einer C₃-C₄-Dihydroxyalkylgruppe, einer C₁-C₄-Aminoalkylgruppe, einer C₁-C₄-Dimethylaminoalkylgruppe, einer C₁-C₄-Acetylaminoalkylgruppe einer C₁-C₄-Methoxyalkylgruppe, einer C₁-C₄-Ethoxyalkylgruppe, einer C₁-C₄-Cyanalkylgruppe, einer C₁-C₄-Carboxyalkylgruppe, einer C₁-C₄-Aminocarbonylalkylgruppe, einer Pyridylmethylgruppe, einer Furfurylgruppe, einer hydrierten Furfurylgruppe, einer substituierten Pyridylgruppe, einem Rest der Formel (II) 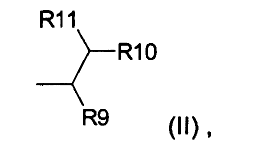 einem Rest der Formel (III) 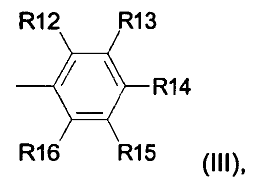 einem Rest der Formel (IV) 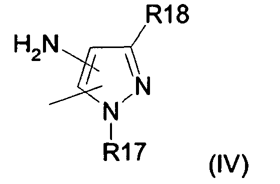 sind oder R6 und R7 gemeinsam einen Ring der Formel 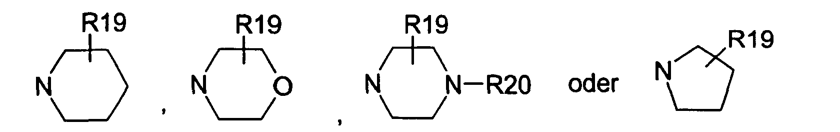 bilden, wobei mindestens einer der Reste R6, R7 kein Wasserstoff ist;
**R8** gleich Wasserstoff, oder einer C₁-C₆ Alkylgruppe ist;
**R9** gleich Wasserstoff, einer Carboxygruppe, oder einer Aminocarbonylgruppe ist;
**R10, R11** unabhängig voneinander gleich Wasserstoff, einer Hydroxygruppe, einer Aminocarbonylgruppe, einer Methylthiomethylgruppe, einem mit einer Phenylgruppe oder Hydroxygruppe substituierten Phenylrest oder einem Rest der Formel 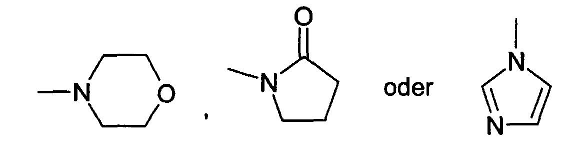 ist ,
**R12,R13,R14,R15,R16** unabhängig voneinander Wasserstoff, ein Halogenatom, eine Cyanogruppe, eine Hydroxygruppe, eine C₁-C₄-Alkoxygruppe, eine C₁-C₄-Hydroxyalkoxygruppe, eine C₁-C₆-Alkylgruppe, eine C₁-C₄-Alkylthioethergruppe, eine Mercaptogruppe, eine Nitrogruppe, eine Aminogruppe, eine Alkylaminogruppe, eine Hydroxyalkylaminogruppe, eine Dialkylaminogruppe, eine Di(hydroxyalkyl)aminogruppe, eine (Dihydroxyalkyl)aminogruppe, eine (Hydroxyalkyl)alkylaminogruppe, eine Trifluormethan-gruppe, eine -C(O)H-Gruppe, eine -C(O)CH₃-Gruppe, eine -C(O)CF₃-Gruppe, eine -Si(CH₃)₃-Gruppe, eine C₁-C₄-Hydroxyalkylgruppe, eine C₃-C₄ Dihydroxyalkylgruppe bedeuten, oder zwei nebeneinanderliegende Reste R12 bis R16 eine -O-CH2-O-Brücke bilden;
**R17** gleich einer C₁-C₄-Alkylgruppe oder einer C₁-C₄-Hydroxyalkylgruppe ist;
**R18** gleich Wasserstoff oder einer C₁-C₆ Alkylgruppe Gruppe ist;
**R19** gleich einer oder mehreren Wasserstoff, oder Hydroxy-, Carboxy-, Aminocarbonyl-, oder Hydroxymethylgruppe ist;
**R20** gleich Wasserstoff, oder einer C₁-C₆ Alkylgruppe Gruppe ist; oder dessen physiologisch verträgliches, wasserlösliches Salz.

2. 2-Aminoalkyl-1,4-diaminobenzol-Derivat nach Anspruch 1, **dadurch gekennzeichnet, dass** in der Formel (I) einer oder mehrere der Reste **R5** bis **R8** gleich Wasserstoff sind.

3. 2-Aminoalkyl-1,4-diaminobenzol-Derivat nach Anspruch 1 oder 2, **dadurch gekennzeichnet, dass** in der Formel (I) die Reste **R1, R2, R3** und **R4** gleich Wasserstoff sind.

4. 2-Aminoalkhyl-1,4-diaminobenzol-Derivat nach einem der Ansprüche 1 bis 3, **dadurch gekennzeichnet, dass** in der Formel (I) **R8** gleich Wasserstoff und **R6** und **R7** unabhängig voneinander gleich Wasserstoff, einer C₁-C₄-Alkylgruppe, einer C₁-C₄-Hydroxyalkylgruppe, einer C₂-C₄-Dihydroxyalkylgruppe,einem Rest der Formel (III) oder (IV) gemäss Anspruch 1 sind oder **R6** und **R7** gemeinsam einen aliphatischen Ring der Formel 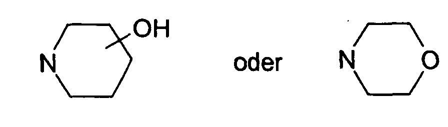 bilden.

5. 2-Aminoalkyl-1,4-diaminobenzol-Derivat nach einem der Ansprüche 1 bis 4, **dadurch gekennzeichnet, dass** es ausgewählt ist aus der Gruppe bestehend aus 2-(2,3-Dihydroxypropyl)aminomethyl-1,4-diamino-benzol; 2-[(2-amino-ethylamino)-methyl]-1,4-diamino-benzol; 2-[(2-hydroxyethylamino)-methyl]-1,4-diamino-benzol; 2-[(2,5-Diamino-benzyl)-methyl-amino]-ethanol; 2-(2,5-Diamino-benzylamino)-propan-1-ol; 2-[(2,5-Diamino-benzyl)-(2-hydroxy-ethyl)-amino]-ethanol; [1-(2,5-Diamino-benzyl)-pyrrolidin-2-yl]-methanol; 1-(2,5-Diamino-benzyl)-pyrrolidin-2-carbonsäureamid; 2-[(4-Methyl-pyridin-2-ylamino)-methyl]-1,4-diamino-benzol; 2-((2-Amino-phenylamino)-methyl)-1,4-diamino-benzol; 2-((2-Chlor-phenylamino)-methyl)-1,4-diamino-benzol; 2-((2-Dimethylamino-phenylamino)-methyl)-1,4-diamino-benzol; 2-((2-Fluor-phenylamino)-methyl)-1,4-diamino-benzol; 2-((2-Hydroxyethylamino-phenylamino)-methyl)-1,4-diamino-benzol; 2-((2-N,N-Bis(hydroxyethyl)-amino-phenylamino)-methyl)-1,4-diamino-benzol; 2-((2-Pyrrolidin-phenylamino)-methyl)-1,4-diamino-benzol; 2-((3-Amino-phenylamino)-methyl)-1,4-diamino-benzol; 2-((3-Chlor-phenylamino)-methyl)-1,4-diamino-benzol; 2-((3-Dimethylamino-phenylamino)-methyl)-1,4-diamino-benzol; 2-((3-Fluor-phenylamino)-methyl)-1,4-diamino-benzol; 2-((3-Hydroxyethylamino-phenylamino)-methyl)-1,4-diamino-benzol; 2-((3-N,N-Bis(hydroxyethyl)amino-phenylamino)-methyl)-1,4-diamino-benzol; 2-((3-Pyrrolidin-phenylamino)-methyl)-1,4-diamino-benzol; 2-((4-Amino-phenylamino)-methyl)-1,4-diamino-benzol; 2-((4-Chlor-phenylamino)-methyl)-1,4-diamino-benzol; 2-((4-Dimethylamino-phenylamino)-methyl)-1,4-diamino-benzol; 2-((4-Fluor-phenylamino)-methyl)-1,4-diamino-benzol; 2-((4-Hydroxyethylamino-phenylamino)-methyl)-1,4-diamino-benzol; 2-((4-N,N-Bis(hydroxyethyl)amino-phenylamino)-methyl)-1,4-diamino-benzol; 2-((4-Pyrrolidin-phenylamino)-methyl)-1,4-diamino-benzol; 2-((2-(2-Hydroxy)-ethoxy-4-amino-phenylamino)-methyl)-1,4-diamino-benzol; 2-((2-Amino-4-amino-phenylamino)-methyl)-1,4-diamino-benzol; 2-((2-Chlor-4-amino-phenylamino)-methyl)-1,4-diamino-benzol; 2-((2-Hydroxy-4-amino-phenylamino)-methyl)-1,4-diamino-benzol; 2-((2-Hydroxyethylamino-4-amino-phenylamino)-methyl)-1,4-diamino-benzol; 2-((2-Methyl-4-amino-phenylamino)-methyl)-1,4-diamino-benzol; 2-((3-(2-Hydroxy)-ethoxy-4-amino-phenylamino)-methyl)-1,4-diamino-benzol; 2-((3-Amino-4-amino-phenylamino)-methyl)-1,4-diamino-benzol; 2-((3-Chlor-4-amino-phenylamino)-methyl)-1,4-diamino-benzol; 2-((3-Hydroxy-4-amino-phenylamino)-methyl)-1,4-diamino-benzol; 2-((3-Hydroxyethylamino-4-amino-phenylamino)-methyl)-1,4-diamino-benzol; 2-((3-Methyl-4-amino-phenylamino)-methyl)-1,4-diamino-benzol; 2-((2-(2-Hydroxy)-ethoxy-phenylamino)-methyl)-1,4-diamino-benzol; 2-((2-Hydroxy-phenylamino)-methyl)-1,4-diamino-benzol; 2-((3-(2-Hydroxy)-ethoxy-phenylamino)-methyl)-1,4-diamino-benzol; 2-((3-Hydroxy-phenylamino)-methyl)-1,4-diamino-benzol; 2-((4-(2-Hydroxy)-ethoxy-phenylamino)-methyl)-1,4-diamino-benzol; 2-((4-Hydroxy-phenylamino)-methyl)-1,4-diamino-benzol; 2-(Phenylamino)-methyl)-1,4-diamino-benzol; 2-[5-Amino-4-(2,5-diamino-phenylamino)-pyrazol-1-yl]-ethanol; N²-(5-Amino-1-methyl-1H-pyrazol-4-yl)-1,2,4-triamino-benzol; N2-(5-Amino-1-isopropyl-1 H-pyrazol-4-yl)-1,2,4-triamino-benzol und N²-(5-Amino-1,3-dimethyl-1H-pyrazol-4-yl)-1,2,4-triamino-benzol.

6. Mittel zum oxidativen Färben von Keratinfasem auf der Basis einer Entwicklersubstanz-Kupplersubstanz-Kombination, **dadurch gekennzeichnet, dass** es als Entwicklersubstanz mindestens ein 2-Aminoalkyl-1,4-diaminobenzol-Derivat der Formel (I) nach einem der Ansprüche 1 bis 5 enthält.

7. Mittel nach Anspruch 6, **dadurch gekennzeichnet, dass** es das 2-Aminoalkyl-1,4-diaminobenzol-Derivat der Formel (I) in einer Menge von 0,005 bis 20,0 Gewichtsprozent enthält.

8. Mittel nach Anspruch 6 oder 7, **dadurch gekennzeichnet, dass** die Kupplersubstanz ausgewählt ist aus der Gruppe bestehend aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxy-ethoxybenzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxybenzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methylphenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxy-propyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

9. Mittel einem der Ansprüche 6 bis 8, **dadurch gekennzeichnet, dass** es außer dem 2-Aminoalkyl-1,4-diaminobenzol-Derivat der Formel (I) zusätzlich mindestens eine weitere Entwicklersubstanz, welche ausgewählt ist aus der Gruppe bestehend aus 1,4-Diaminobenzol, 2,5-Diaminotoluol, 2,5-Diaminophenylethylalkohol, 4-Aminophenol und seinen Derivaten, 4,5-Diaminopyrazolderivaten und Tetraaminopyrimidinen, enthält.

10. Mittel nach einem der Ansprüche 6 bis 9, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

11. Mittel nach einem der Ansprüche 6 bis 10 , **dadurch gekennzeichnet, dass** es zusätzlich mindestens einen direktziehenden Farbstoff enthält.

12. Mittel nach einem der Ansprüche 6 bis 11, **dadurch gekennzeichnet, daß** es ein Haarfärbemittel ist.

## Claims

1. 2-Aminoalkyl-1,4-diaminobenzene derivative of the general formula (I) 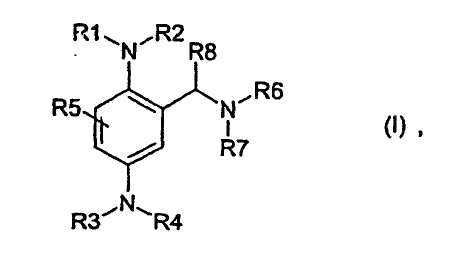 in which
**R1, R2, R3** and **R4,** independently of one another, are hydrogen, a C₁-C₆-alkyl group, a C₁-C₄-hydroxyalkyl group, a C₂-C₄-dihydroxyalkyl group or a C₁-C₄-alkoxy-(C₁-C₂)-alkyl group or R1 and R2 form a four-membered to eight-membered aliphatic ring, where at least two of the radicals R1 to R4 are hydrogen;
**R5** is hydrogen, a halogen atom, a C₁-C₄-alkyl group, a C₁-C₄-hydroxyalkyl group or a C₁-C₄-alkoxy group;
**R6 and R7,** independently of one another, are hydrogen, a C₁-C₂-alkoxy group, a C₁-C₆-alkyl group, an unsaturated C₁-C₆-alkyl group, a C₁-C₉-hydroxyalkyl group, a C₃-C₄-dihydroxyalkyl group, a C₁-C₄-aminoalkyl group, a C₁-C₄-dimethylaminoalkyl group, a C₁-C₄-acetylaminoalkyl group, a C₁-C₄-methoxyalkyl group, a C₁-C₄-ethoxyalkyl group, a C₁-C₄-cyanoalkyl group, a C₁-C₄-carboxyalkyl group, a C₁-C₄-aminocarbonylalkyl group, a pyridylmethyl group, a furfuryl group, a hydrogenated furfuryl group, a substituted pyridyl group, a radical of the formula (II) 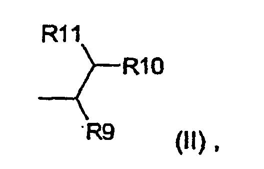 a radical of the formula (III) 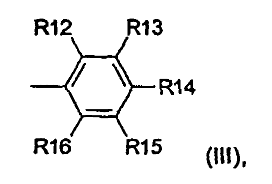 a radical of the formula (IV) 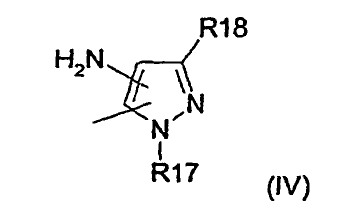 or R6 and R7 together form a ring of the formula 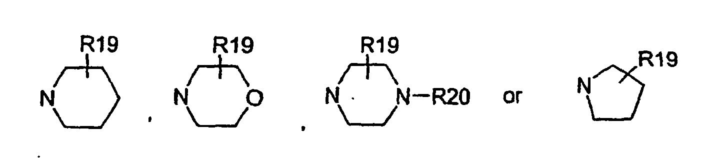 where at least one of the radicals R6, R7 is not hydrogen;
**R8** is hydrogen, or a C₁-C₆-alkyl group;
**R9** is hydrogen, a carboxy group or an aminocarbonyl group;
**R10, R11**, independently of one another, are hydrogen, a hydroxyl group, an aminocarbonyl group, a methylthiomethyl group, a phenyl radical substituted by a phenyl group or hydroxyl group or a radical of the formula 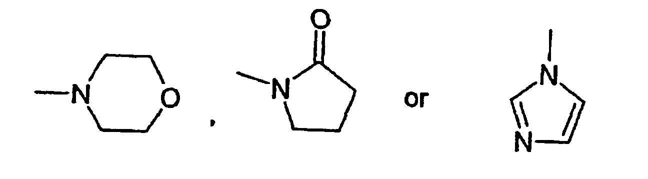 **R12, R13, R14, R15, R16**, independently of one another, are hydrogen, a halogen atom, a cyano group, a hydroxy group, a C₁-C₄-alkoxy group, a C₁-C₄-hydroxyalkoxy group, a C₁-C₆-alkyl group, a C₁-C₄-alkyl thioether group, a mercapto group, a nitro group, an amino group, an alkylamino group, a hydroxyalkylamino group, a dialkylamino group, a di(hydroxyalkyl)amino group, a (dihydroxyalkyl)amino group, a (hydroxyalkyl)alkylamino group, a trifluoromethane group, a -C(O)H group, a -C (O) CH₃ group, a -C(O)CF₃ group, an -Si (CH₃)₃ group, a C₁-C₄-hydroxyalkyl group, a C₃-C₄-dihydroxyalkyl group, or two adjacent radicals R12 to R16 form a -O-CH2-O bridge;
**R17** is a C₁-C₄-alkyl group or a C₁-C₄-hydroxyalkyl group;
**R18** is hydrogen or a C₁-C₆-alkyl group;
**R19** is one or more hydrogen, or hydroxyl, carboxy, aminocarbonyl or hydroxymethyl group;
**R20** is hydrogen, or a C₁-C₆-alkyl group; or physiologically compatible, water-soluble salt thereof.

2. 2-Aminoalkyl-1,4-diaminobenzene derivative according to Claim 1, **characterized in that**, in the formula (I), one or more of the radicals **R5** to **R8** are hydrogen.

3. 2-Aminoalkyl-1,4-diaminobenzene derivative according to Claim 1 or 2, **characterized in that**, in the formula (I), the radicals **R1, R2, R3** and **R4** are hydrogen.

4. 2-Aminoalkyl-1,4-diaminobenzene derivative according to one of Claims 1 to 3, **characterized in that**, in the formula (I), **R8** is hydrogen and **R6** and **R7,** independently of one another, are hydrogen, a C₁-C₄-alkyl group, a C₁-C₄-hydroxyalkyl group, a C₂-C₄-dihydroxyalkyl group, a radical of the formula (III) or (IV) according to Claim 1, or **R6** and **R7** together form an aliphatic ring of the formula

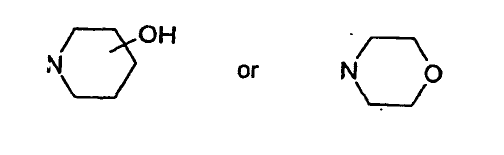

5. 2-Aminoalkyl-1,4-diaminobenzene derivative according to one of Claims 1 to 4, **characterized in that** it is chosen from the group consisting of 2-(2,3-dihydroxypropyl)aminomethyl-1,4-diaminobenzene; 2-[(2-aminoethylamino)methyl]-1,4-diaminobenzene; 2-[(2-hydroxyethylamino)methyl]-1,4-diaminobenzene; 2-[(2,5-diaminobenzyl)methylamino]ethanol; 2-(2,5-diaminobenzylamino)propan-1-ol; 2-[(2,5-diaminobenzyl)-(2-hydroxyethyl)amino]ethanol; [1-(2,5-diaminobenzyl)pyrrolidin-2-yl]methanol; 1-(2,5-diaminobenzyl)pyrrolidine-2-carboxamide; 2-[(4-methylpyridin-2-ylamino)methyl]-1,4-diaminobenzene; 2-((2-aminophenylamino)methyl)-1,4-diaminobenzene; 2-((2-chlorophenylamino)methyl)-1,4-diaminobenzene; 2-((2-dimethylaminophenylamino)methyl)-1,4-diaminobenzene; 2-((2-fluorophenylamino)methyl-1,4-diaminobenzene; 2-((2-hydroxyethylaminophenylamino)methyl)-1,4-diaminobenzene; 2-((2-N,N-bis(hydroxyethyl)aminophenylamino)methyl)-1,4-diaminobenzene; 2-((2-pyrrolidinphenylamino)methyl)-1,4-diaminobenzene; 2-((3-aminophenylamino)methyl)-1,4-diaminobenzene; 2-((3-chlorophenylamino)methyl)-1,4-diaminobenzene; 2-((3-dimethylaminophenylamino)methyl)-1,4-diaminobenzene; 2-((3-fluorophenylamino)methyl)-1,4-diaminobenzene: 2-((3-hydroxyethylaminophenylamino)methyl)-1,4-diaminobenzene; 2-((3-N,N-bis(hydroxyethyl)aminophenylamino)methyl)-1,4-diaminobenzene; 2-((3-pyrrolidinphenylamino)methyl)-1,4-diaminobenzene; 2-((4-aminophenylamino)methyl)-1,4-diaminobenzene; 2-((4-chlorophenylamino)methyl)-1,4-diaminobenzene; 2-((4-dimethylaminophenylamino)methyl)-1,4-diaminobenzene; 2-((4-fluorophenylamino)methyl)-1,4-diaminobenzene; 2-((4-hydroxyethylaminophenylamino)methyl)-1,4-diaminobenzene; 2-((4-N,N-bis(hydroxyethyl)aminophenylamino)methyl)-1,4-diaminobenzene; 2-((4-pyrrolidinphenylamino)methyl)-1,4-diaminobenzene; 2-((2-(2-hydroxy)ethoxy-4-aminophenylamino)methyl)-1,4-diaminobenzene; 2-((2-amino-4-aminophenylamino)methyl)-1,4-diaminobenzene; 2-((2-chloro-4-aminophenylamino)methyl)-1,4-diaminobenzene: 2-((2-hydroxy-4-aminophenylamino)methyl)-1,4-diaminobenzene; 2-((2-hydroxyethylamino-4-aminophenylamino)methyl)-1,4-diaminobenzene; 2-((2-methyl-4-aminophenylamino)methyl)-1,4-diaminobenzene; 2-((3-(2-hydroxy)ethoxy-4-aminophenylamino)methyl)-1,4-diaminobenzene; 2-((3-amino-4-aminophenylamino)methyl)-1,4-diaminobenzene; 2-((3-chloro-4-aminophenylamino)methyl)-1,4-diaminobenzene; 2-((3-hydroxy-4-aminophenylamino)methyl)-1,4-diaminobenzene; 2-((3-hydroxyethylamino-4-aminophenylamino)methyl)-1,4-diaminobenzene; 2-((3-methyl-4-aminophenylamino)methyl)-1,4-diaminobenzene; 2-((2-(2-hydroxy)ethoxyphenylamino)methyl)-1,4-diaminobenzene; 2-((2-hydroxyphenylamino)methyl)-1,4-diaminobenzene; 2-((3-(2-hydroxy)ethoxyphenylamino)methyl)-1,4-diaminobenzene; 2-((3-hydroxyphenylamino)methyl)-1,4-diaminobenzene; 2-((4-(2-hydroxy)ethoxyphenylamino)methyl)-1,4-diaminobenzene; 2-((4-hydroxyphenylamino)methyl)-1,4-diaminobenzene; 2-(phenylamino)methyl)-1,4-diaminobenzene; 2-[5-amino-4-(2,5-diaminophenylamino)pyrazol-1-yl]ethanol; N²-(5-amino-1-methyl-1H-pyrazol-4-yl)-1,2,4-triaminobenzene; N²-(5-amino-1-isopropyl-1H-pyrazol-4-yl)-1,2,4-triaminobenzene and N²-(5-amino-1,3-dimethyl-1H-pyrazol-4-yl)-1,2,4-triaminobenzene.

6. Composition for the oxidative dyeing of keratin fibres based on a developer substance-coupler substance combination, **characterized in that** it comprises, as developer substance, at least one 2-aminoalkyl-1,4-diaminobenzene derivative of the formula (I) according to one of Claims 1 to 5.

7. Composition according to Claim 6, **characterized in that** it comprises the 2-aminoalkyl-1,4-diaminobenzene derivative of the formula (I) in an amount of from 0.005 to 20.0% by weight.

8. Composition according to Claim 6 or 7, **characterized in that** the coupler substance is chosen from the group consisting of 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylamino-phenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]-acetamide, 5-[(2-hydroxyethyl)amino]-2-methylphenol, 3-[(2-hyroxyethyl)amino]phenol, 3-[(2-methoxyethyl)amino]phenol, 5-amino-2-ethylphenol, 2-(4-amino-2-hydroxyphenoxy) ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methylphenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

9. Composition according to one of Claims 6 to 8, **characterized in that**, apart from the 2-aminoalkyl-1,4-diaminobenzene derivative of the formula (I), it additionally comprises at least one further developer substance which is chosen from the group consisting of 1,4-diaminobenzene, 2,5-diaminotoluene, 2,5-diaminophenylethyl alcohol, 4-aminophenol and its derivatives, 4,5-diaminopyrazole derivatives and tetraaminopyrimidines.

10. Composition according to one of Claims 6 to 9, **characterized in that** the developer substances and coupler substances, based on the total amount of the dye composition, are in each case present in a total amount of from 0.005 to 20% by weight.

11. Composition according to one of Claims 6 to 10, **characterized in that** it additionally comprises at least one direct dye.

12. Composition according to one of Claims 6 to 11, **characterized in that** it is a hair dyeing composition.

## Revendications

1. Dérivé de 2-aminoalkyl-1,4-diaminobenzène de formule générale (I) 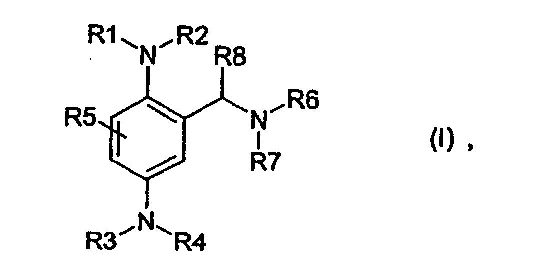 dans laquelle
**R1, R2, R3** et **R4** représentent indépendamment les uns des autres un atome d'hydrogène, un groupe alkyle en C₁-C₆, un groupe hydroxyalkyle en C₁-C₄, un groupe dihydroxyalkyle en C₂-C₄ ou un groupe alcoxy (C₁-C₄)-alkyle (C₁-C₂)_{'} ou R1 et R2 forment un cycle aliphatique à 4 à 8 chaînons, au moins 2 des radicaux R1 à R4 représentant un atome d'hydrogène ;
**R5** représente un atome d'hydrogène, un atome d'halogène, un groupe alkyle en C₁-C₄, un groupe hydroxyalkyle en C₁-C₄ ou un groupe alcoxy en C₁-C₄ ;
**R6** et **R7** représentent, indépendamment l'un de l'autre, un atome d'hydrogène, un groupe alcoxy en C₁-C₂, un groupe alkyle en C₁-C₆, un groupe alkyle en C₁-C₆ insaturé, un groupe hydroxyalkyle en C₁-C₄, un groupe dihydroxyalkyle en C₃-C₄, un groupe aminoalkyle en C₁-C₄, un groupe diméthylaminoalkyle-(C₁-C₄), un groupe acétylaminoalkyle (C₁-C₄), un groupe méthoxyalkyle (C₁-C₄), un groupe éthoxyalkyle (C₁-C₄), un groupe cyanoalkyle (C₁-C₄), un groupe carboxyalkyle (C₁-C₄), un groupe aminocarboxylalkyle(C₄-C₄), un groupe pyridylméthyle, un groupe furfuryle, un groupe furfuryle hydrogéné, un groupe pyridyle substitué, un radical de formule (II) 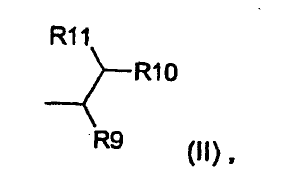 un radical de formule (III) 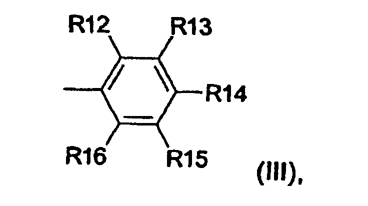 un radical de formule (IV) 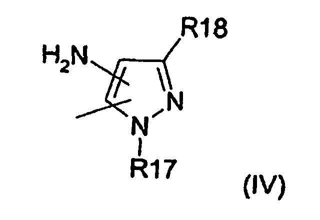 ou R6 et R7 forment ensemble un cycle de formule 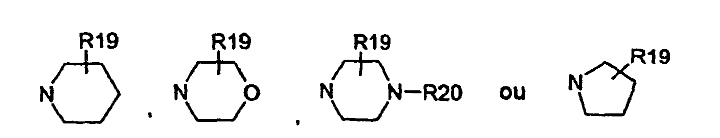 au moins l'un des radicaux R6, R7 n'étant pas un atome d'hydrogène ;
**R8** représente un atome d'hydrogène ou un groupe alkyle en C₁-C₆ ;
**R9** représente un atome d'hydrogène, un groupe carboxy ou un groupe aminocarbonyle ;
**R10, R11** représentent indépendamment l'un de l'autre un atome d'hydrogène, un groupe hydroxy, un groupe aminocarbonyle, un groupe méthylthiométhyle, un radical phényle substitué par un groupe phényle ou par un groupe hydroxy, ou un radical de formule 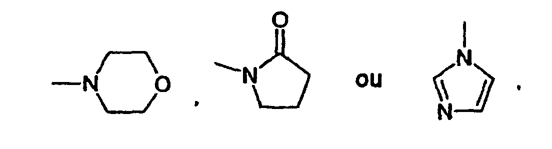 **R12, R13, R14, R15, R16** représentent, indépendamment les uns des autres, un atome d'hydrogène, un atome d'halogène, un groupe cyano, un groupe hydroxy, un groupe alcoxy en C₁-C₄, un groupe hydroxyalcoxy en C₁-C₄, un groupe alkyle en C₁-C₆, un groupe alkyl(C₁-C₄)thioéther, un groupe mercapto, un groupe nitro, un groupe amino, un groupe alkylamino, un groupe hydroxyalkylamino, un groupe dialkylamino, un groupe di(hydroxyalkyl)amino, un groupe (dihydroxyalkyl)amino, un groupe (hydroxyalkyl)alkylamino, un groupe trifluorométhane, un groupe -C(O)H, un groupe -C(O)CH₃, un groupe -C(O)CF₃, un groupe -Si(CH₃)₃, un groupe hydroxyalkyle en C₁-C₄, un groupe dihydroxyalkyle en C₃-C₄, ou deux radicaux R12 à R16 contigus forment un pont -O-CH₂-O- ;
**R17** représente un groupe alkyle en C₁-C₄ ou un groupe hydroxyalkyle en C₁-C₄ ;
**R18** représente un atome d'hydrogène ou un groupe alkyle en C₁-C₆ ;
**R19** représente un ou plusieurs atomes d'hydrogène ou un groupe hydroxy, carboxy, aminocarbonyle ou hydroxyméthyle ;
**R20** représente un atome d'hydrogène ou un groupe alkyle en C₁-C₆ ;
ou sel hydrosoluble physiologiquement acceptable d'un tel dérivé.

2. Dérivé de 2-aminoalkyl-1,4-diaminobenzène selon la revendication 1, **caractérisé en ce que** dans la formule (I) un ou plusieurs des radicaux **R5** à **R8** représentent un atome d'hydrogène.

3. Dérivé de 2-aminoalkyl-1,4-diaminobenzène selon la revendication 1 ou 2, **caractérisé en ce que** dans la formule (I) les radicaux **R1, R2, R3** et **R4** représentent un atome d'hydrogène.

4. Dérivé de 2-aminoalkyl-1,4-diaminobenzène selon l'une quelconque des revendications 1 à 3, **caractérisé en ce que** dans la formule (I) **R8** représente un atome d'hydrogène et **R6** et **R7** représentent, indépendamment l'un de l'autre, un atome d'hydrogène, un groupe alkyle en C₁-C₄, un groupe hydroxyalkyle en C₁-C₄, un groupe dihydroxyalkyle en C₂-C₄, un radical de formule (III) ou (IV) selon la revendication 1, ou **R6** et **R7** forment ensemble un cycle aliphatique de formule

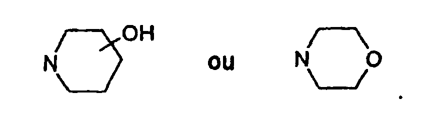

5. Dérivé de 2-aminoalkyl-1,4-diaminobenzène selon l'une quelconque des revendications 1 à 4, **caracté** **risé en ce qu'**il est choisi dans le groupe constitué par le 2-(2,3-dihydroxypropyl)aminométhyl-1,4-diaminobenzène, le 2-[(2-aminoéthylamino)méthyl]-1,4-diaminobenzène, le 2-[(2-hydroxyéthylamino)-méthyl]-1,4-diaminobenzène, le 2-[(2,5-diaminobenzyl)méthylamino]éthanol, le 2-(2,5-diaminobenzylamino)propan-1-ol, le 2-[(2,5-diaminobenzyl)-(2-hydroxyéthyl)amino]éthanol, le [1-(2,5-diaminobenzyl)pyrrolidin-2-yl]méthanol, le 1-(2,5-diaminobenzyl)pyrrolidine-2-carboxamide, le 2-[(4-méthylpyridin-2-ylamino)méthyl]-1,4-diaminobenzène, le 2-((2-aminophénylamino)méthyl)-1,4-diaminobenzène, le 2-((2-chlorophénylamino)méthyl)-1,4-diaminobenzène, le 2-((2-diméthylaminophénylamino)méthyl-1,4-diaminobenzène, le 2-((2-fluorophénylamino)-méthyl)-1,4-diaminobenzène, le 2-((2-hydroxyéthylaminophénylamino)méthyl)-1,4-diaminobenzène, le 2-((2-N,N-bis(hydroxyéthyl)aminophénylamino)méthyl)-1,4-diaminobenzène, le 2-((2-pyrrolidine-phénylamino)méthyl)-1,4-diaminobenzène, le 2-((3-aminophénylamino)méthyl)-1,4-diaminobenzène, le 2-((3-chlorophénylamino)méthyl)-1,4-diaminobenzène, le 2-((3-diméthylaminophénylamino)méthyl)-1,4-diaminobenzène, le 2-((3-fluorophénylamino)méthyl)-1,4-diaminobenzène, le 2-((3-hydroxyéthylaminophénylamino)méthyl)-1,4-diaminobenzène, le 2-((3-N,N-bis(hydroxyéthyl)aminophénylamino)méthyl)-1,4-diaminobenzène, le 2-((3-pyrrolidine-phénylamino)-méthyl)-1,4-diaminobenzène, le 2-((4-aminophénylamino)méthyl)-1,4-diaminobenzène, le 2-((4-chorophénylamino)méthyl)-1,4-diaminobenzène, le 2-((4-diméthylaminophénylamino)méthyl)-1,4-diaminobenzène, le 2-((4-fluorophénylamino)méthyl)-1,4-diaminobenzène, le 2-((4-hydroxyéthylaminophénylamino)méthyl)-1,4-diaminobenzène, le 2-((4-N,N-bis(hydroxyéthyl)aminophénylamino)méthyl)-1,4-diaminobenzène, le 2-((4-pyrrolidine-phénylamino)-méthyl)-1,4-diaminobenzène, le 2-((2-(2-hydroxy)-éthoxy-4-aminophénylamino)méthyl)-1,4-diaminobenzène, le 2-((2-amino-4-aminophénylamino)méthyl)-1,4-diaminobenzène, le 2-((2-chloro-4-aminophénylamino)méthyl)-1,4-diaminobenzène, le 2-((2-hydroxy-4-aminophénylamino)méthyl)-1,4-diaminobenzène, le 2-((2-hydroxyéthylamino-4-aminophénylamino)méthyl)-1,4-diaminobenzène, le 2-((2-méthyl-4-aminophénylamino)méthyl)-1,4-diaminobenzène, le 2-((3-(2-hydroxy)éthoxy-4-aminophénylamino)méthyl)-1,4-diaminobenzène, le 2-((3-amino-4-aminophénylamino)-méthyl)-1,4-diaminobenzène, le 2-((3-chloro-4-aminophénylamino)méthyl)-1,4-diaminobenzène, le 2-((3-hydroxy-4-aminophénylamino)méthyl)-1,4-diaminobenzène, le 2-((3-hydroxyéthylamino-4-aminophénylamino)méthyl)-1,4-diaminobenzène, le 2-((3-méthyl-4-aminophénylamino)méthyl)-1,4-diaminobenzène, le 2-((2-(2-hydroxy)éthoxyphénylamino)méthyl)-1,4-diaminobenzène, le 2-((2-hydroxyphénylamino)méthyl)-1,4-diaminobenzène, le 2-((3-(2-hydroxy)éthoxyphénylamino)méthyl)-1,4-diaminobenzène, le 2-((3-hydroxyphénylamino)-méthyl)-1,4-diaminobenzène, le 2-((4-(2-hydroxy)-éthoxyphénylamino)méthyl)-1,4-diaminobenzène, le 2-((4-hydroxyphénylamino)méthyl)-1,4-diaminobenzène, le 2-(phénylamino)méthyl-1,4-diaminobenzène, le 2-[5-amino-4-(2,5-diaminophénylamino)-pyrazol-1-yl]éthanol, le N²-(5-amino-1-méthyl-1H-pyrazol-4-yl)-1,2,4-triaminobenzène, le N²-(5-amino-1-isopropyl-1H-pyrazol-4-yl)-1,2,4-triaminobenzène et le N²-(5-amino-1,3-diméthyl-1H-pyrazol-4-yl)-1,2,4-triaminobenzène.

6. Composition pour la teinture par oxydation de fibres de kératine, à base d'une association développeur-coupleur, **caractérisée en ce qu'**elle contient en tant que développeur au moins un dérivé de 2-aminoalkyl-1,4-diaminobenzène de formule (I) selon l'une quelconque des revendications 1 à 5.

7. Composition selon la revendication 6, **caractérisée en ce qu'**elle contient le dérivé de 2-aminoalkyl-1,4-diaminobenzène de formule (I) en une quantité de 0,005 à 20,0 % en poids.

8. Composition selon la revendication 6 ou 7, **caractérisée en ce que** le développeur est choisi dans le groupe constitué par la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)-pyridine, la 2,6-diamino-3,5-diméthoxypyridine, la 3,5-diamino-2,6-diméthoxypyridine, le 1,3-diaminobenzène, le 2,4-diamino-1-(2-hydroxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxyacétique, la 3-[di(2-hydroxyéthyl)amino]aniline, le 4-amino-2-di[(2-hydroxyéthyl)amino]-1-éthoxybenzène, le 5-méthyl-2-(1-méthyléthyl)phénol, la 3-[(2-hydroxyéthyl)amino]-aniline, la 3-[(2-aminoéthyl)amino]aniline, le 1,3-di(2,4-diaminophénoxy)propane, le di(2,4-diaminophénoxy)méthane, le 1,3-diamino-2,4-diméthoxybenzène, le 2,6-bis(2-hydroxyéthyl)aminotoluène, le 4-hydroxy-indole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxyphényl)amino]-acétamide, le 5-[(2-hydroxyéthyl)amino]-2-méthylphénol, le 3-[(2-hydroxyéthyl)amino]phénol, le 3-[(2-méthoxyéthyl)amino]phénol, le 5-amino-2-éthylphénol, le 2-(4-amino-2-hydroxyphénoxy)-éthanol, le 5-[(3-hydroxypropyl)amino]-2-méthylphénol, le 3-[(2,3-dihydroxypropyl)amino]-2-méthylphénol, le 3-[(2-hydroxyéthyl)amino]-2-méthylphénol, la 2-amino-3-hydroxypyridine, le 5-amino-4-chloro-2-méthylphénol, le 1-naphtol, le 1,5-dihydroxynaphthalène, le 1,7-dihydroxynaphthalène, le 2,3-dihydroxynaphthalène, le 2,7-dihydroxynaphthalène, l'acétate de 2-méthyl-1-naphtol, le 1,3-dihydroxybenzène, le 1-chloro=2,4-dihydroxybenzène, le 2-chloro-1,3-dihydroxybenzène, le 1,2-dichloro-3,5-dihydroxy-4-méthylbenzène, le 1,5-dichloro-2,4-dihydroxybenzène, le 1,3-dihydroxy-2-méthylbenzène, le 3,4-méthylènedioxyphénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxy-indole, la 5,6-dihydroxy-indoline, le 5-hydroxy-indole, le 6-hydroxy-indole, le 7-hydroxy-indole et la 2,3-indolinedione.

9. Composition selon l'une quelconque des revendications 6 à 8, **caractérisée en ce que**, outre le dérivé de 2-aminoalkyl-1,4-diaminobenzène de formule (I), elle contient additionnellement au moins un autre développeur qui est choisi dans le groupe constitué par le 1,4-diaminobenzène, le 2,5-diaminotoluène, l'alcool 2,5-diaminophényléthylique, le 4-aminophénol et ses dérivés, des dérivés de 4,5-diaminopyrazole et des tétraaminopyrimidines.

10. Composition selon l'une quelconque des revendications 6 à 9, **caractérisée en ce que** les développeurs et les coupleurs sont contenus chacun en une quantité totale de 0,005 à 20 % en poids, par rapport à la quantité totale de la composition de teinture.

11. Composition selon l'une quelconque des revendications 6 à 10, **caractérisée en ce qu'**elle contient additionnellement au moins un colorant direct.

12. Composition selon l'une quelconque des revendications 6 à 11, **caractérisée en ce qu'**il s'agit d'une composition de teinture pour cheveux.
